# EUROPEAN PATENT APPLICATION

(11) **EP 4 567 818 A1**
(43) Date of publication of application: **11.06.2025**
(21) Application number: 23862500.8
(22) Date of filing: 08.09.2023
(51) Int. Cl.: G16B 30/10

(54) **METHOD FOR DESIGNING HUMANIZED ANTIBODY SEQUENCE**

(30) Priority: 09.09.2022 CN 202211101597
(71) Applicant: Nanjing GenScript Biotech Co., Ltd., Nanjing, Jiangsu 211100 (CN); Genscript (Shanghai) Biotech Co., Ltd., Shanghai 200131 (CN)
(72) Inventor: FAN, Long, Shanghai 200131 (CN); HE, Yuzhuo, Nanjing, Jiangsu 211100 (CN); HAO, Xiaohu, Nanjing, Jiangsu 211100 (CN); LI, Gen, Shanghai 200131 (CN); DING, Lidan, Nanjing, Jiangsu 211100 (CN)
(74) Representative: Wang, Bo
(86) International application number: PCT/CN2023/117671
(87) International publication number: WO 2024/051806

(57) **Abstract**

Provided in the present application is a method for designing a humanized antibody sequence. The method may comprise: determining a variable region and a CDR region of an initial antibody; acquiring a human-derived variable region sequence template from a database containing a plurality of human-derived variable region sequences on the basis of the sequence of the variable region of the initial antibody; substituting the sequence of the CDR region in the human-derived variable region sequence template into the sequence of the CDR region of the initial antibody to obtain a target variable region sequence template; executing iterative simulated evolution by means of using a genetic algorithm on the basis of the target variable region sequence template to determine a plurality of candidate variable region sequences; determining a plurality of candidate humanized antibody sequences and/or a plurality of candidate humanized antibody functional fragment sequences on the basis of the plurality of candidate variable region sequences; and determining one or more target humanized antibody sequences and/or target humanized antibody functional fragment sequences from the plurality of candidate humanized antibody sequences and/or the plurality of candidate humanized antibody functional fragment sequences.

## Description

### CROSS-REFERENCE TO RELATED APPLICATION

**The** present invention claims priority to Chinese Patent Application No. 202211101597.X filed on September 9, 2022, the entire contents of which are incorporated herein by reference.

### TECHNICAL FIELD

**The** present description relates to the field of antibody engineering and, in particular, to a method for designing a humanized antibody sequence.

### BACKGROUND

Antibodies can specifically recognize antigens and are widely used in the fields of biopharmaceuticals and immunotherapy, such as cancer therapy, immunoregulatory disease therapy and infectious disease therapy. The safety of antibodies is one of the most important factors affecting antibody applications. The immunogenicity of non-human-derived antibodies not only causes a decrease in the safety of antibody drugs, but also stimulates the body to produce neutralizing antibodies, resulting in a loss of antibody activity. Thus, in practical applications, humanization processing can be performed on a non-human-derived antibody to modify the antibody sequence, such that the non-human-derived antibody is closer to the human antibody sequence, thereby reducing the immunogenicity of the antibody and enhancing the safety of the antibody drug. Specifically, antibody humanization is a process of substituting a part of a sequence of a non-human-derived antibody with a sequence of a human-derived antibody, such that the sequence of the non-human-derived antibody is more like that of the human-derived antibody and has reduced or eliminated immunogenicity. Currently, antibody humanization mainly comprises the following methods: a chimerization method, CDR grafting, SDR grafting, a resurfacing method, germ cell lineage humanization based on CDR or SDR grafting, etc. Traditional antibody humanization processing relies primarily on manual operations, such as empirical rational design and back mutation site analysis. Such an approach is highly dependent on the experience and knowledge level of the researcher and has limited success in designing humanized antibody sequences. If the affinity of the obtained humanized antibody does not meet the requirements, it is required to analyze and design the humanized antibody sequence again, which will take a lot of time and effort. Therefore, it is necessary to provide a more efficient method for designing a humanized antibody sequence.

### SUMMARY

According to an aspect of the present description, a method for designing a humanized antibody sequence is provided by one or more embodiments. The method may comprise: performing sequence analysis on a sequence of an initial antibody to determine a variable region and a CDR region of the initial antibody; acquiring a human-derived variable region sequence template from a database containing a plurality of human-derived variable region sequences on the basis of the sequence of the variable region of the initial antibody; substituting the sequence of the CDR region in the human-derived variable region sequence template into the sequence of the CDR region of the initial antibody to obtain a target variable region sequence template; executing iterative simulated evolution by means of using a genetic algorithm on the basis of the target variable region sequence template to determine a plurality of candidate variable region sequences; determining a plurality of candidate humanized antibody sequences and/or a plurality of candidate humanized antibody functional fragment sequences on the basis of the plurality of candidate variable region sequences; and determining one or more target humanized antibody sequences and/or target humanized antibody functional fragment sequences from the plurality of candidate humanized antibody sequences and/or the plurality of candidate humanized antibody functional fragment sequences.

In some embodiments, the executing iterative simulated evolution by means of using a genetic algorithm on the basis of the target variable region sequence template to determine a plurality of candidate variable region sequences comprises: executing a simulated mutation on a framework region of the target variable region sequence template according to a preset mutation probability on the basis of the target variable region sequence template to obtain a plurality of initial variable region sequences; predicting a humanization probability of each of the plurality of initial variable region sequences using a trained depth forest model; executing an iterative simulated evolution using the genetic algorithm on the basis of the plurality of initial variable region sequences and the humanization probability to determine a plurality of candidate variable region sequences.

In some embodiments, the executing an iterative simulated evolution using the genetic algorithm on the basis of the plurality of initial variable region sequences and the humanization probability to determine a plurality of candidate variable region sequences comprises: determining, for each of the plurality of initial variable region sequences, a distance between the initial variable region sequence and the target variable region sequence template, and determining an individual fitness of the initial variable region sequence on the basis of the distance between the initial variable region sequence and the target variable region sequence template and the humanization probability of the initial variable region sequence; executing an iterative simulated evolution on the basis of the plurality of initial variable region sequences and the individual fitness to determine a plurality of candidate variable region sequences.

In some embodiments, the individual fitness is determined using an NGSA-II algorithm or a derivative algorithm thereof.

In some embodiments, the target variable region sequence template comprises a target heavy chain variable region sequence template and a target light chain variable region sequence template, and the plurality of candidate variable region sequences comprises a plurality of candidate heavy chain variable region sequences and a plurality of candidate light chain variable region sequences.

In some embodiments, the executing a simulated mutation on a framework region of the target variable region sequence template according to a preset mutation probability on the basis of the target variable region sequence template to obtain a plurality of initial variable region sequences comprises: executing a simulated mutation on a framework region of the first variable region sequence template according to the preset mutation probability on the basis of a first variable region sequence template corresponding to a first variable region in the target variable region sequence template to obtain a plurality of initial populations corresponding to the first variable region, wherein each of the plurality of initial populations comprises the plurality of initial variable region sequences corresponding to the first variable region, and the first variable region is a heavy chain variable region or a light chain variable region.

In some embodiments, each simulated evolution operation in the iterative simulated evolution comprises: selecting a plurality of initial variable region sequences from the plurality of initial variable region sequences as a plurality of first sequences on the basis of the individual fitness of each of the plurality of initial variable region sequences corresponding to the first variable region; retaining the plurality of first sequences of the plurality of initial populations and removing unselected initial variable region sequences of the plurality of initial populations to obtain a plurality of first populations; executing a simulated migration among the plurality of first populations to obtain a plurality of second populations, each of the plurality of second populations comprising a plurality of second sequences; executing a simulated crossover and a simulated variation on the plurality of second sequences of the plurality of second populations to obtain a plurality of third populations, each of the plurality of third populations comprising a plurality of third sequences; determining one or more first candidate variable region sequences from the plurality of third sequences.

In some embodiments, a termination condition for the iterative simulated evolution comprises at least one of the following conditions: a total number of rounds of the iterative simulated evolution being greater than or equal to a first threshold; a total number of first candidate variable region sequences obtained by the iterative simulated evolution being greater than or equal to a second threshold; a diversity of the one or more first candidate variable region sequences obtained by a current round of simulated evolution operation being less than or equal to a third threshold; an average similarity of first candidate variable region sequences obtained by a current round of simulated evolution operation to first candidate variable region sequences obtained by a previous round of simulated evolution operation being greater than or equal to a fourth threshold.

In some embodiments, the first variable region sequence template comprises a first template and/or a second template, wherein the first template is a human-derived variable region sequence which is acquired from the database containing a plurality of human-derived variable region sequences and has the highest similarity to a sequence of a framework region of a first variable region of the initial antibody, and the second template is a human-derived variable region sequence which is acquired from the database containing a plurality of human-derived variable region sequences and has the highest similarity to a complete sequence of a first variable region of the initial antibody.

In some embodiments, the first variable region sequence template comprises the first template and the second template; the plurality of initial populations comprises a plurality of first initial populations obtained by executing a simulated mutation on the basis of the first template and a plurality of second initial populations obtained by executing a simulated mutation on the basis of the second template; the plurality of first populations comprises a plurality of first retained populations obtained by retaining the first sequences from the first initial populations and a plurality of second retained populations obtained by retaining the first sequences from the second initial populations.

In some embodiments, the executing a simulated migration among the plurality of first populations to obtain a plurality of second populations, comprises: executing a simulated migration among the plurality of first retained populations, executing a simulated migration among the plurality of second retained populations, and executing a simulated migration between the first retained populations and the second retained populations to obtain the plurality of second populations.

In some embodiments, the trained deep forest model is obtained by the following steps: acquiring a plurality of training samples corresponding to the first variable region, wherein each of the plurality of training samples comprises a human-derived or non-human-derived variable region sequence and a tag indicating that the sample variable region sequence is human-derived or non-human-derived, and the sample variable region sequence is subjected to length standardization processing; training an initial deep forest model using the plurality of training samples to obtain a trained deep forest model.

In some embodiments, the determining a plurality of candidate humanized antibody sequences and/or a plurality of candidate humanized antibody functional fragment sequences on the basis of the plurality of candidate variable region sequences comprises: determining a plurality of selected candidate heavy chain variable region sequences from the plurality of candidate heavy chain variable region sequences; determining a plurality of selected candidate light chain variable region sequences from the plurality of candidate light chain variable region sequences; determining the plurality of candidate humanized antibody sequences and/or the plurality of candidate humanized antibody functional fragment sequences on the basis of the plurality of selected candidate heavy chain variable region sequences and the plurality of selected candidate light chain variable region sequences.

In some embodiments, the performing sequence analysis on a sequence of an initial antibody to determine a variable region and a CDR region in the sequence of the initial antibody, comprises: performing sequence analysis on a sequence of an initial antibody to determine a heavy chain variable region, a light chain variable region, a heavy chain CDR region, and a light chain CDR region of the initial antibody; performing length standardization processing on a sequence of the heavy chain variable region; and performing length standardization processing on a sequence of the light chain variable region.

According to another aspect of the present description, another method for designing a humanized antibody sequence is provided by one or more embodiments. The method comprises: performing sequence analysis on a sequence of an initial antibody to determine a variable region and a CDR region of the initial antibody; acquiring a human-derived variable region sequence template from a database containing a plurality of human-derived variable region sequences on the basis of the sequence of the variable region of the initial antibody; substituting the sequence of the CDR region in the human-derived variable region sequence template into the sequence of the CDR region of the initial antibody to obtain a target variable region sequence template; executing a simulated mutation on a framework region of the target variable region sequence template according to a preset mutation probability on the basis of the target variable region sequence template to obtain a plurality of initial variable region sequences; predicting a humanization probability of each of the plurality of initial variable region sequences using a trained depth forest model; determining a plurality of candidate humanized antibody sequences and/or a plurality of candidate humanized antibody functional fragment sequences on the basis of the plurality of initial variable region sequences and the humanization probability; determining one or more target humanized antibody sequences and/or target humanized antibody functional fragment sequences from the plurality of candidate humanized antibody sequences or functional fragments thereof.

In some embodiments, the determining a plurality of candidate humanized antibody sequences and/or a plurality of candidate humanized antibody functional fragment sequences on the basis of the plurality of initial variable region sequences and the humanization probability comprises: determining a plurality of candidate humanized antibody sequences and/or a plurality of candidate humanized antibody functional fragment sequences using a heuristic algorithm on the basis of the plurality of initial variable region sequences and the humanization probability.

In some embodiments, the heuristic algorithm comprises at least one of the following algorithms: a genetic algorithm, a particle swarm algorithm, an ant colony algorithm, a simulated annealing method, a list search algorithm, evolutionary programming, evolutionary strategy and neural networks.

In some embodiments, the determining a plurality of candidate humanized antibody sequences and/or a plurality of candidate humanized antibody functional fragment sequences using a heuristic algorithm on the basis of the plurality of initial variable region sequences and the humanization probability comprises: executing an iterative simulated evolution using a genetic algorithm on the basis of the plurality of initial variable region sequences and the humanization probability to determine a plurality of candidate variable region sequences; determining the plurality of candidate humanized antibody sequences and/or the plurality of candidate humanized antibody functional fragment sequences on the basis of the plurality of candidate variable region sequences.

In some embodiments, the executing an iterative simulated evolution using a genetic algorithm on the basis of the plurality of initial variable region sequences and the humanization probability to determine a plurality of candidate variable region sequences comprises: determining, for each of the plurality of initial variable region sequences, a distance between the initial variable region sequence and the target variable region sequence template, and determining an individual fitness of the initial variable region sequence on the basis of the distance between the initial variable region sequence and the target variable region sequence template and the humanization probability of the initial variable region sequence; executing an iterative simulated evolution on the basis of the plurality of initial variable region sequences and the individual fitness to determine a plurality of candidate variable region sequences.

In some embodiments, the individual fitness is determined using an NGSA-II algorithm or a derivative algorithm thereof.

In some embodiments, the target variable region sequence template comprises a target heavy chain variable region sequence template and a target light chain variable region sequence template, and the plurality of candidate variable region sequences comprises a plurality of candidate heavy chain variable region sequences and a plurality of candidate light chain variable region sequences.

In some embodiments, the executing a simulated mutation on a framework region of the target variable region sequence template according to a preset mutation probability on the basis of the target variable region sequence template to obtain a plurality of initial variable region sequences comprises: executing a simulated mutation on a framework region of the first variable region sequence template according to the preset mutation probability on the basis of a first variable region sequence template corresponding to a first variable region in the target variable region sequence template to obtain a plurality of initial populations corresponding to the first variable region, wherein each of the plurality of initial populations comprises the plurality of initial variable region sequences corresponding to the first variable region, and the first variable region is a heavy chain variable region or a light chain variable region.

In some embodiments, each simulated evolution operation in the iterative simulated evolution comprises: selecting a plurality of initial variable region sequences from the plurality of initial variable region sequences as a plurality of first sequences on the basis of the individual fitness of each of the plurality of initial variable region sequences corresponding to the first variable region; retaining the plurality of first sequences of the plurality of initial populations and removing unselected initial variable region sequences of the plurality of initial populations to obtain a plurality of first populations; executing a simulated migration among the plurality of first populations to obtain a plurality of second populations, each of the plurality of second populations comprising a plurality of second sequences; executing a simulated crossover and a simulated variation on the plurality of second sequences of the plurality of second populations to obtain a plurality of third populations, each of the plurality of third populations comprising a plurality of third sequences; determining one or more first candidate variable region sequences from the plurality of third sequences.

In some embodiments, a termination condition for the iterative simulated evolution comprises at least one of the following conditions: a total number of rounds of the iterative simulated evolution being greater than or equal to a first threshold; a total number of first candidate variable region sequences obtained by the iterative simulated evolution being greater than or equal to a second threshold; a diversity of the one or more first candidate variable region sequences obtained by a current round of simulated evolution operation being less than or equal to a third threshold; an average similarity of first candidate variable region sequences obtained by a current round of simulated evolution operation to first candidate variable region sequences obtained by a previous round of simulated evolution operation being greater than or equal to a fourth threshold.

In some embodiments, the first variable region sequence template comprises a first template and/or a second template, wherein the first template is a human-derived variable region sequence which is acquired from the database containing a plurality of human-derived variable region sequences and has the highest similarity to a sequence of a framework region of a first variable region of the initial antibody, and the second template is a human-derived variable region sequence which is acquired from the database containing a plurality of human-derived variable region sequences and has the highest similarity to a complete sequence of a first variable region of the initial antibody.

In some embodiments, the first variable region sequence template comprises the first template and the second template; the plurality of initial populations comprises a plurality of first initial populations obtained by executing a simulated mutation on the basis of the first template and a plurality of second initial populations obtained by executing a simulated mutation on the basis of the second template; the plurality of first populations comprises a plurality of first retained populations obtained by retaining the first sequences from the first initial populations and a plurality of second retained populations obtained by retaining the first sequences from the second initial populations.

In some embodiments, the executing a simulated migration among the plurality of first populations to obtain a plurality of second populations, comprises: executing a simulated migration among the plurality of first retained populations, executing a simulated migration among the plurality of second retained populations, and executing a simulated migration between the first retained populations and the second retained populations to obtain the plurality of second populations.

In some embodiments, the determining a plurality of candidate humanized antibody sequences and/or a plurality of candidate humanized antibody functional fragment sequences on the basis of the plurality of candidate variable region sequences comprises: determining a plurality of selected candidate heavy chain variable region sequences from the plurality of candidate heavy chain variable region sequences; determining a plurality of selected candidate light chain variable region sequences from the plurality of candidate light chain variable region sequences; determining the plurality of candidate humanized antibody sequences and/or the plurality of candidate humanized antibody functional fragment sequences on the basis of the plurality of selected candidate heavy chain variable region sequences and the plurality of selected candidate light chain variable region sequences.

In some embodiments, the trained deep forest model is obtained by the following steps: acquiring a plurality of training samples corresponding to the first variable region, wherein each of the plurality of training samples comprises a human-derived or non-human-derived variable region sequence and a tag indicating that the sample variable region sequence is human-derived or non-human-derived, and the sample variable region sequence is subjected to length standardization processing; training an initial deep forest model using the plurality of training samples to obtain a trained deep forest model.

In some embodiments, the performing sequence analysis on a sequence of an initial antibody to determine a variable region and a CDR region in the sequence of the initial antibody, comprises: performing sequence analysis on a sequence of an initial antibody to determine a heavy chain variable region, a light chain variable region, a heavy chain CDR region, and a light chain CDR region of the initial antibody; performing length standardization processing on a sequence of the heavy chain variable region; and performing length standardization processing on a sequence of the light chain variable region.

According to yet another aspect of the present description, a computing device is provided by one or more embodiments. The computing device comprises at least one processor and at least one storage device, wherein the at least one storage device has stored thereon an instruction set used for designing a humanized antibody sequence, and the instruction set, when executed, causes the processor to execute the method for designing a humanized sequence as described above.

According to yet another aspect of the present description, a non-transitory computer-readable storage medium is provided by one or more embodiments, wherein, the non-transitory computer-readable storage medium has stored thereon an instruction set used for designing a humanized antibody sequence, and the instruction set, when executed by a computing device, causes the computing device to execute the method described above involving a humanized sequence.

### BRIEF DESCRIPTION OF THE DRAWINGS

**The** present description will be further illustrated by way of exemplary embodiments, and these exemplary embodiments will be described in detail with reference to the accompanying drawings. These embodiments are not restrictive, and in these embodiments, the same numerals represent the same structures, in which:
FIG. 1 is a method for designing a humanized antibody sequence shown according to some embodiments of the present description;
FIG. 2 is a method for obtaining a plurality of candidate variable region sequences using a genetic algorithm shown according to some embodiments of the present description;
FIG. 3 is a method for obtaining a plurality of candidate variable region sequences using a genetic algorithm shown according to some embodiments of the present description;
FIG. 4 is a method for designing a humanized antibody sequence shown according to some embodiments of the present description;
FIG. 5 is a method for determining one or more target humanized antibodies on the basis of a plurality of candidate variable region sequences shown according to some embodiments of the present description;
FIG. 6 is a schematic diagram of an exemplary computing device shown according to some embodiments of the present description.

### DETAILED DESCRIPTION

**As** shown in the present description and the claims, the words "one", "a", "an" and/or "the" do not specifically refer to the singular, but may also comprise the plural, unless the context clearly indicates otherwise. Generally, the terms "comprising" and "comprising" only imply the inclusion of explicitly identified steps and elements, and these steps or elements do not constitute an exclusive list. A method or a device may further comprise other steps or elements.

Unless otherwise defined, all technical and scientific terms used herein have the same meaning as commonly understood by one of ordinary skill in the art to which the present disclosure pertains.

The terms "about" and "around" may describe a certain range of values around a particular value, such as plus or minus 10%, 9%, 8%, 7%, 6%, 5%, 4%, 3%, 2%, or 1% of the value. For example, the term "about 10 mL" may comprise 9 mL to 11 mL.

Embodiments of the present description involve a method for designing a humanized antibody. In some embodiments, the method may comprise performing sequence analysis on a sequence of an initial antibody using a computing device to determine a variable region and a complementarity determining region (CDR region) in the sequence of the initial antibody; determining a human-derived variable region sequence template from a database containing a plurality of human-derived variable region sequences on the basis of a sequence of the variable region of the initial antibody; substituting the sequence of the CDR region in the human-derived variable region sequence template into the sequence of the CDR region of the initial antibody to obtain a target variable region sequence template; executing iterative simulated evolution by means of using a genetic algorithm on the basis of the target variable region sequence template to determine a plurality of candidate variable region sequences; determining a plurality of candidate humanized antibody sequences on the basis of the plurality of candidate variable region sequences; determining one or more target humanized antibody sequences from the plurality of candidate humanized antibody sequences. In some embodiments, the target variable region sequence template comprises a heavy chain variable region sequence template and a light chain variable region sequence template, and the plurality of candidate variable region sequences comprises a plurality of candidate heavy chain variable region sequences and a plurality of candidate light chain variable region sequences.

In some embodiments, the method can be performed using a computing device, so as to efficiently obtain a plurality of candidate humanized sequences. For example, a computing device can employ a genetic algorithm to simulate a process of natural evolution, and can obtain a plurality of candidate humanized antibody sequences after humanization processing on the basis of a target variable region sequence template by means of a simulated mutation, a population migration, a simulated crossover and a simulated variation, thereby providing more choices for the humanization application of antibodies. For the obtained candidate humanized antibody sequences, various methods can also be employed for further screening. For example, the properties of the candidate humanized antibodies in aspects such as the solubility, post-translational modification sites, antigen-antibody molecule docking, and protein stability are evaluated by a computing device, and the candidate humanized antibody sequences after screening are obtained according to the results of the evaluation. For the candidate humanized antibody sequences after screening, candidate humanized antibodies can be synthesized and tested for properties in various aspects, such as affinity of the antibody, the expression efficiency of the antibody and the stability of the antibody, by chemical or biological experiments. On the basis of the results of the experiments, the candidate humanized antibody with properties meeting the requirements can be selected as the target humanized antibody.

**As** used herein, the term "computing device" may refer to a class of devices having information processing capabilities, comprising at least one processor and at least one storage device. The processor is used for executing various information processing processes, such as for executing a genetic algorithm, to obtain a plurality of candidate humanized antibody sequences after humanization processing. The storage device is used for storing various information and data and may also store instructions that may be run in the processor to execute the steps in the method for designing a humanized antibody sequence provided in some embodiments of the present description. In some embodiments, the computing device may further comprise or be connected to a terminal device. The terminal device may comprise an input device such as a mouse or a keyboard. The terminal device may further comprise an output device such as a screen or a speaker. The user can interact with the processor and/or storage device via the terminal device, for example, can view and control the process of designing the humanized antibody sequence, view the obtained candidate humanized sequence, etc.

It should be understood that the application scenarios of the present description are merely some examples or embodiments of the present description, and for those of ordinary skill in the art, it would also have been possible to apply the present description to other similar scenarios according to these accompanying drawings without making creative efforts.

**The** methods involved in the embodiments of the present description would be described in detail below in conjunction with the accompanying drawings. It should be noted that the following embodiments are only for illustrating the present description and do not constitute any limitation of the present description.

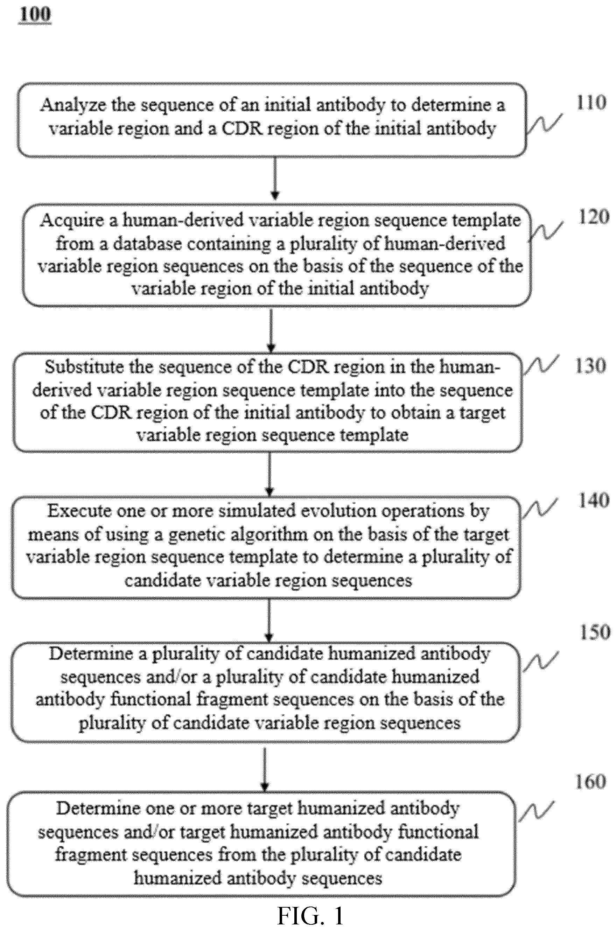

FIG. 1 is a method for designing a humanized antibody sequence shown according to some embodiments of the present description. In some embodiments, a process 100 comprises at least step 110 to step 160. In some embodiments, at least a part of steps 110-160 may be executed on a computing device, such as a computing device 600.

In step 110, sequence analysis may be performed on a sequence of an initial antibody to determine a variable region and a CDR region in the sequence of the initial antibody.

In some embodiments, the initial antibody may be an antibody derived from any non-human species, e.g., from mice, rats, rabbits, monkeys, chimpanzees, dogs, cats, etc. By means of steps 110-150, a plurality of target antibody sequences and/or a plurality of target antibody functional fragment sequences after humanization processing may be obtained on the basis of the initial antibody. The plurality of target antibody sequences or target antibody functional fragment sequences have a relatively high similarity (e.g., greater than 80%) to human antibody sequences or antibody functional fragment sequences, have higher safety, and can also better maintain the functional activity of the antibody.

In some embodiments, various suitable methods may be adopted to perform sequence analysis on the sequence of the initial antibody. For example, corresponding antibody annotation software (e.g., ANARCI) may be used on a computing device to perform position annotation on the sequence of the initial antibody and determine the variable region and the CDR region in the initial antibody. By way of example only, amino acids at various sites in the initial antibody may be aligned and numbered according to the international Immunogenetics information system (IMGT) rules.

In some embodiments, the heavy chain variable region sequence and light chain variable region sequence after being numbered may also be subjected to length standardization processing, so as to facilitate the comparison of the heavy chain variable region sequence and the light chain variable region sequence with the standardized sequence template in subsequent steps, or to determine the humanization probabilities of the heavy chain variable region sequence and the light chain variable region sequence. The length standardization processing refers to the modification of the lengths of the heavy chain variable region sequence and the light chain variable region sequence to the standardized length of the heavy chain variable region and the standardized length of the light chain variable region, respectively, by filling in blank positions in the sequence after alignment.

In some embodiments, a deep learning model may be used in the process of designing a humanized antibody sequence to predict the humanization probability of a sequence (see, e.g., the relevant content in step 140 about using a deep forest model to predict the humanization probability of a sequence). The training set of the deep learning model comprises humanized and non-humanized antibody variable region sequences acquired from a database. The length standardization processing is also required for the human-derived and non-human-derived antibody variable region sequences. For example, the heavy and light chain variable region sequences of human-derived and non-human-derived antibodies may be acquired from databases such as OAS and SAbDab, divided into two groups by the heavy chain and the light chain, and then subjected to coordinate alignment and length standardization processing according to the amino acid sequences within the group, respectively, such as after numbering and alignment according to IMGT rules, where blank positions of the sequences may be filled with the character "X". After the length standardization processing, all sequences within the heavy chain group have the same length (i.e., the standardized length of the heavy chain variable region) and all sequences within the light chain group have the same length (i.e., the standardized length of the light chain variable region). In some embodiments, the standardized length of the heavy chain variable region sequence and the standardized length of the light chain variable region sequence may be the same or different. In some embodiments, the sequence of the initial antibody variable region may also be subjected to the length standardization processing according to the same method, such that the length of the heavy chain variable region sequence of the initial antibody is consistent with the standardized length of the heavy chain variable region in the training set, and the length of the light chain variable region sequence of the initial antibody is consistent with the standardized length of the light chain variable region in the training set.

In step 120, a human-derived variable region sequence template may be obtained from a database containing a plurality of human-derived variable region sequences on the basis of a sequence of the variable region of the initial antibody.

In some embodiments, the computing device may acquire a database containing a plurality of human-derived variable region sequences from a storage device or an external device. The database may contain variable region sequences derived from various human-derived antibodies, comprising heavy chain variable region sequences and light chain variable region sequences. In some embodiments, the heavy chain variable region sequences and light chain variable region sequences in the database are also subjected to length standardization processing. The length of the heavy chain variable region sequence in the database may be the same as the length of the heavy chain variable region sequence of the initial antibody in step 110 after the length standardization processing. Similarly, the length of the light chain variable region sequence in the database may be the same as the length of the light chain variable region sequence of the initial antibody in step 110 after the length standardization processing.

In some embodiments, the human-derived heavy chain variable region sequence template and the human-derived light chain variable region sequence template may be determined from the database according to the heavy chain variable region sequence and light chain variable region sequence of the initial antibody. By way of example only, the CDR regions in the heavy chain variable region sequence of the initial antibody may be replaced with the mask "X", the sequence of the framework region (FR region) is retained, and then a humanized heavy chain variable region template with the highest similarity to the sequence of the framework region in the heavy chain variable region of the initial antibody is found after searching from the database and used as a first template. For another example, a humanized heavy chain variable region template having the highest similarity to a complete sequence of the heavy chain variable region of the initial antibody can be found after searching from the database and is used as a second template. In some embodiments, for a heavy chain variable region sequence, only a first template or a second template may be determined, or both the first template and the second template may be determined. For a light chain variable region sequence, the first template and/or the second template corresponding to the light chain variable region may be determined in a similar manner, which will not be repeated here.

In step 130, a sequence of a CDR region in the human-derived variable region sequence template may be substituted with a sequence of the CDR region of the initial antibody to obtain a target variable region sequence template.

In some embodiments, the process of substituting the sequence of the CDR region in the human-derived variable region sequence template with the sequence of the CDR region of the initial antibody, also referred to as CDR grafting, can enable the target variable region sequence template to retain the affinity of the initial antibody for the antigen as much as possible. The target variable region sequence template may comprise a target heavy chain variable region sequence template and a target light chain variable region sequence template. Specifically, the CDR region in the human-derived heavy chain variable region template may be substituted with the CDR region of the heavy chain variable region of the initial antibody to obtain the target heavy chain variable region template. Similarly, the CDR region in the human-derived light chain variable region template may be substituted with the CDR region of the light chain variable region of the initial antibody to obtain the target light chain variable region template.

In step 140, an iterative simulated evolution may be executed on the basis of the target variable region sequence template using a genetic algorithm to determine a plurality of candidate variable region sequences.

In some embodiments, an iterative simulated evolution is required to be performed on the two initial populations using a genetic algorithm on the basis of an initial population corresponding to the heavy chain variable region and an initial population corresponding to the light chain variable region to determine a plurality of candidate heavy chain variable region sequences and candidate light chain variable region sequences. Since the process of acquiring the candidate heavy chain variable region sequence is similar to that of acquiring the candidate light chain variable region sequence, for ease of description, a "first variable region" is used herein to refer to a heavy chain variable region or a light chain variable region. In some embodiments, a simulated mutation may be executed on a framework region of a first variable region sequence template according to a preset mutation probability to obtain a plurality of initial populations corresponding to the first variable region, wherein each of the plurality of initial populations comprises a plurality of initial variable region sequences. On the basis of the plurality of initial populations, the iterative simulated evolution may be executed until a termination condition is reached, so as to obtain a plurality of candidate variable region sequences corresponding to the first variable region.

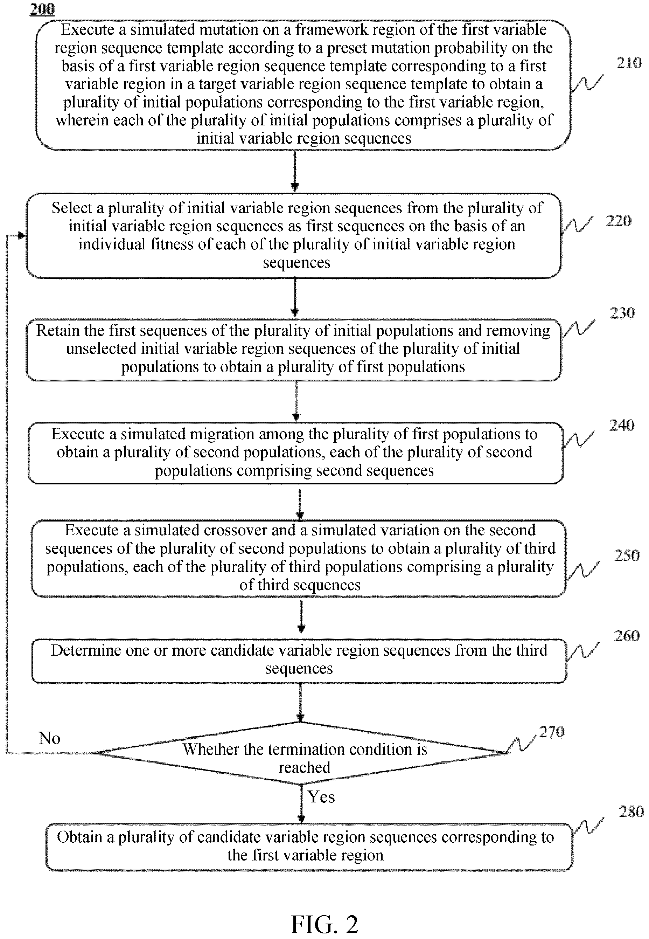

In some embodiments, the basic iterative process of the genetic algorithm may comprise the following steps: selecting suitable parent sequences, recombining the parent sequences to obtain progeny sequences, selecting an optimized individual from the progeny sequences and retaining same, and repeating this process until a termination condition is reached, such that a plurality of optimized individuals can be obtained. Each iteration is considered to be a process of the simulated evolution. Specifically, each iterative process in the iterative simulated evolution may comprise the following steps: (1) selecting a plurality of initial variable region sequences from the plurality of initial variable region sequences as first sequences on the basis of the individual fitness of each of the plurality of initial variable region sequences; (2) retaining the first sequences of the plurality of initial populations and removing unselected initial variable region sequences of the plurality of initial populations to obtain a plurality of first populations; (3) executing a simulated migration among the plurality of first populations to obtain a plurality of second populations, each of the plurality of second populations comprising a second sequence; (4) executing a simulated crossover and a simulated variation on the second sequences of the plurality of second populations to obtain a plurality of third populations, each of the plurality of third populations comprising a plurality of third sequences; (5) determining one or more first candidate variable region sequences from the third sequences. Steps (1)-(3) are equivalent to searching for a suitable parent individual (referring to a specific sequence), step (4) is a process of recombining the parent sequences to obtain progeny individuals, and step (5) is a process of screening for optimized individuals from the obtained progeny individuals and retaining the same. For more information on the simulated mutation, simulated migration and simulated crossover, see other sections of the description, e.g., the description in FIG. 2.

In some embodiments, when a genetic algorithm is used, a user may check and adjust genetic algorithm-related parameters. For example, the computing device 600 may present preset genetic algorithm-related parameters to a user through an output device or other terminal devices, and the user may check and adjust those parameters. For example, the genetic algorithm-related parameters may comprise: the probability of mutation, the number of initial populations, the number of individuals in the initial population (i.e., initial variable region sequences), the probability of crossover of individuals in the population, and the probability of migration between populations. In some embodiments, the user may also check and adjust the termination condition, such as a total number of rounds of iterative simulated evolution. By way of example only, the probability of mutation may be about 0.01, 0.02 or 0.05; the number of initial populations may be 2, 3, 5 or 10; the number of individuals in the initial population may be about 100, 200 or 500; the probability of crossover of individuals in the population may be about 0.7, 0.8 or 0.85; the probability of migration between populations may be 0.005, 0.01 or 0.02. In some embodiments, the values of the genetic algorithm-related parameters adopted to acquire the candidate heavy chain variable region and the values of the genetic algorithm-related parameters adopted to acquire the light chain variable region may be identical, partially identical or completely different.

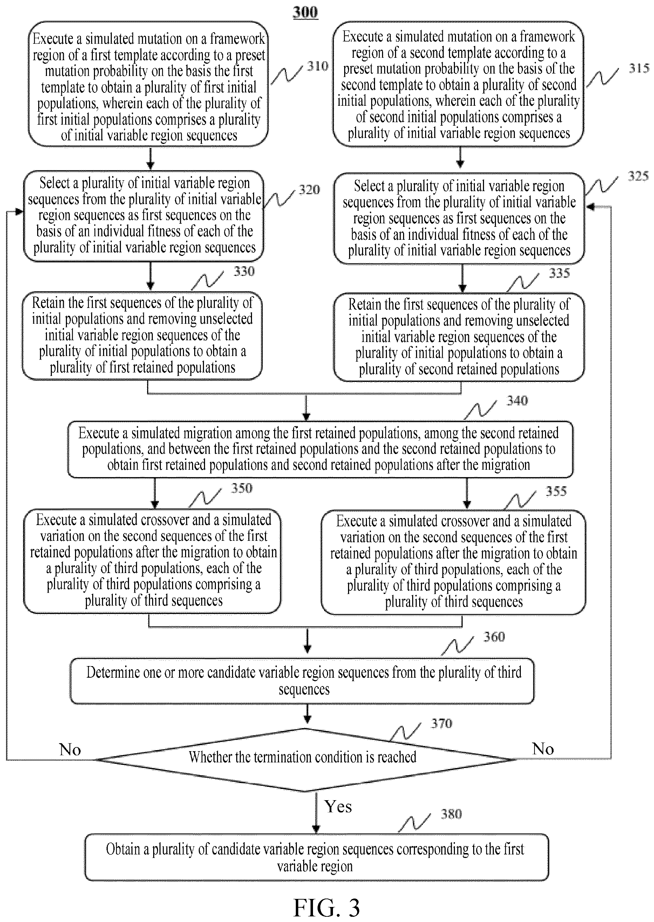

In some embodiments, the genetic algorithm used may be a single-population genetic algorithm or a multi-population genetic algorithm. The single-population genetic algorithm means that when determining a plurality of candidate variable region sequences corresponding to the first variable region by iterative simulated evolution, one initial population is generated on the basis of only one first variable region sequence template (e.g., a first template or a second template in step 120), and no subsequent migration between the populations is performed. However, the multi-population genetic algorithm can generate a plurality of initial populations, each containing a plurality of initial variable region sequences. In some embodiments, the genetic algorithm may use one template or a plurality of templates corresponding to the first variable region. For example, the genetic algorithm may use a first template and a second template simultaneously to separately generate a plurality of initial populations, and optimized individuals are separately screened to obtain two types of first populations corresponding to the two templates. When migration is performed between first populations, the migration can be performed between the same type of first populations (i.e., corresponding to the same template) or between different types of first populations (i.e., corresponding to different templates). For more details on the process of performing iterative simulated evolution using the multi-population genetic algorithm, see other sections of the description, e.g., FIG. 2, FIG. 3 and related descriptions thereof.

In some embodiments, a termination condition for the iterative simulated evolution may comprise one or more of the following conditions: a total number of times of the iterative simulated evolution being greater than or equal to a first threshold; a total number of first candidate variable region sequences obtained by the iterative simulated evolution being greater than or equal to a second threshold; a diversity of the one or more first candidate variable region sequences obtained by a current round of simulated evolution operation being less than or equal to a third threshold; an average similarity of first candidate variable region sequences obtained by a current round of simulated evolution operation to first candidate variable region sequences obtained by a previous round of simulated evolution operation being greater than or equal to a fourth threshold.

In some embodiments, the individual fitness may be determined according to a humanization probability and a distance between the initial variable region sequence and the first variable region sequence template using an NGSA-II algorithm or a derivative algorithm thereof. In some embodiments, the distance between the initial variable region sequence and the first variable region sequence template may be an edit distance, also referred to as a Levenshtein distance. The degree of difference between two strings can be quantified by calculating the minimum number of operations required to convert one string to the other, thereby obtaining the edit distance. In some embodiments, the distance between the initial variable region sequence and the first variable region sequence template may be a distance calculated on the basis of a substitution scoring matrix of protein sequence alignment, such as a point accepted mutation (PAM) matrix and a predicted hydrophobic and transmembrane (PHAT) matrix. After optimization by the genetic algorithm, compared with the initialization template, the output initial variable region sequence will achieve an increase in the predicted humanization probability as large as possible with the sequence changes or distance increase being minimized.

In some embodiments, a trained deep learning model may be used for predicting a humanization probability of an individual sequence. In some embodiments, the trained deep learning model may be a trained deep forest model. The advantages of using the deep forest over other deep learning models are as follows: (1) the deep forest has more stable and good learning performance when processing data of different sizes, that is, it is not demanding on data size, features a rapid training speed, and reduces computational costs; (2) compared with other deep learning models (e.g., deep neural networks), there is no need to set complex hyper-parameters, and the corresponding parameters are automatically set by minimizing the training error on specific datasets, that is, the modeling difficulty is reduced; (3) the tree structure of the deep forest has better interpretability than neural networks, that is, it is conducive to mining and interpreting deep biological meanings. As used herein, the term "humanization probability" is an indicator used for measuring the degree of humanization, which may be a numerical value between 0 and 1, and may be expressed as a numerical value such as 0.7 or 0.8, or expressed as a percentage such as 70% or 80%. The initial variable region sequence may be input to a trained deep learning model that may output the humanization probability of the initial variable region sequence.

In some embodiments, a plurality of training samples corresponding to the first variable region (comprising a heavy chain variable region and a light chain variable region) may be acquired, wherein each of the plurality of training samples comprises a sample variable region sequence and a tag indicating that the sample variable region sequence is human-derived or non-human-derived. For example, the tag may be 0 or 1. When the tag is 0, it indicates that the sample variable region sequence is a non-human-derived sequence; when the tag is 1, it indicates that the sample variable region sequence is a human-derived sequence. Alternatively, when the tag is 1, it indicates that the sample variable region sequence is a human-derived sequence; when the tag is 0, it indicates that the sample variable region sequence is a non-human-derived sequence. The sample variable region sequence is also subjected to length standardization processing to ensure that the sizes of various sample variable region sequences remain consistent when converted to a feature vector, which facilitates inputting into the deep learning model for processing. After acquiring a plurality of training samples, an initial deep learning model (such as a deep forest model) may be trained to obtain a trained deep learning model. In some embodiments, a deep forest model used for predicting a humanization probability for a heavy chain variable region sequence may be obtained by training using training samples corresponding to the heavy chain variable region; a deep forest model used for predicting a humanization probability for a light chain variable region sequence may be obtained by training using training samples corresponding to the light chain variable region. In some embodiments, the training samples may be derived from a variety of species, and the deep forest models obtained after training have a relatively high adaptability and can be used for predicting the humanization probabilities for a variety of species. In some embodiments, a plurality of training samples corresponding to a species may be used depending on the species, and the obtained deep forest models may be dedicated to predicting the humanization probabilities for the variable region sequences of the species.

In some specific embodiments, the deep learning model is a deep forest model, and constructing the deep forest model comprising the following steps: (1) dividing heavy and light chain variable region sequence samples of human-derived and non-human-derived antibodies acquired from the database into two groups by the heavy chain and the light chain, and unifying the lengths of the sequences within the heavy and light chain groups according to the method of the length standardization processing of the heavy and light chain variable regions described in step 110; (2) performing deep forest model training on the length-standardized variable region sequence samples in heavy chain group and the length-standardized variable region sequence samples in the light chain group in step (1), to obtain a deep forest model used for predicting the humanization probabilities for the heavy chain variable region sequences and a deep forest models used for predicting the humanization probabilities for the light chain variable region sequences, respectively.

In step 150, a plurality of candidate humanized antibody sequences and/or a plurality of candidate humanized antibody functional fragment sequences may be determined on the basis of the plurality of candidate variable region sequences.

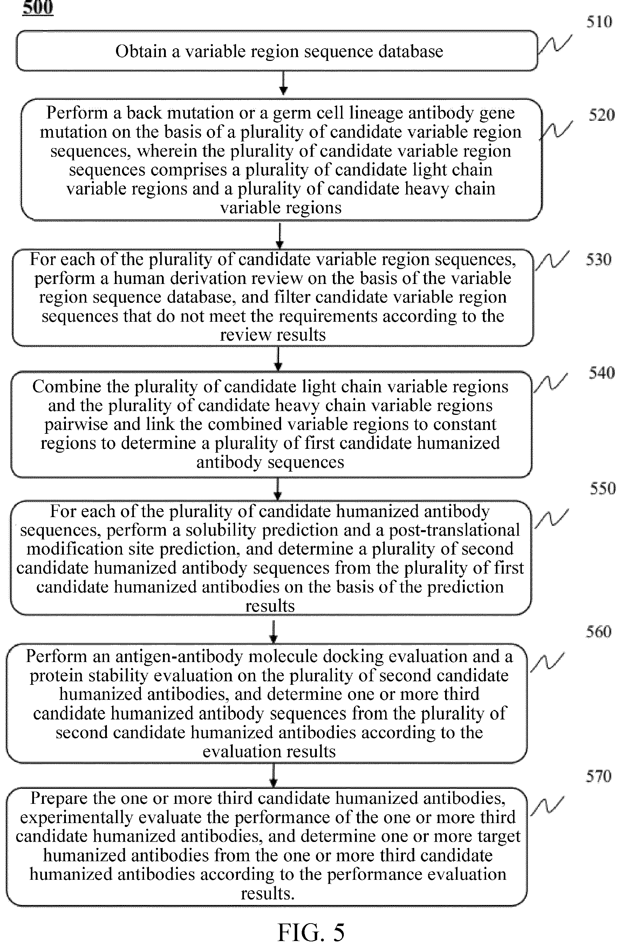

In some embodiments, the candidate variable region sequences may be further screened by various methods. For example, a human derivation review may be performed on the candidate heavy chain variable region sequences and the candidate light chain variable region sequences on the basis of a variable region sequence database, and candidate heavy chain variable region sequences and candidate light chain variable region sequences that meet the requirements may be selected according to the review results. The variable region sequence database may comprise various human-derived and non-human-derived variable region sequences, for example, any published database containing antibody information, such as OAS, SAbDab, and NCBI database, or a combination thereof. The variable region sequence database may be searched for a variable region sequence that has the highest similarity to a certain candidate variable region sequence, and if the variable region sequence that has the highest similarity is a human-derived sequence, the sequence is considered to meet the requirements. Alternatively, a back mutation or a germ cell lineage antibody gene mutation may also be performed before the human derivation review. For more details on screening candidate variable region sequences, see FIG. 5 and the description thereof.

In some embodiments, by the screening manner described above, a plurality of selected candidate heavy chain variable region sequences may be determined from the plurality of candidate heavy chain variable region sequences; a plurality of selected candidate light chain variable region sequences may be determined from the plurality of candidate light chain variable region sequences. In some embodiments, selected candidate light chain variable region sequences and selected candidate heavy chain variable region sequences may also be determined on the basis of the humanization probability. The humanization probability may be predicted using the deep forest model described above. A human-derived heavy chain constant region may be added to the plurality of selected candidate heavy chain variable region sequences to obtain a plurality of candidate humanized heavy chain sequences; a human-derived light chain constant region may be added to the plurality of selected candidate light chain variable region sequences to obtain a plurality of candidate humanized light chain sequences. For example, the human-derived constant regions may be the heavy chain constant region and the light chain constant region of an IgG1 after designing or screening. Further, the plurality of candidate humanized heavy chain sequences and the plurality of candidate humanized light chain sequences may be combined pairwise to determine the plurality of candidate humanized antibody sequences. In some embodiments, candidate humanized heavy chain sequences and candidate humanized light chain sequences may be randomly selected to be combined to form candidate humanized antibody sequences. Alternatively, candidate light chain variable region sequences and candidate heavy chain variable region sequences that have relatively high humanization probabilities may also be selected to be combined to form candidate humanized antibody sequences.

In some embodiments, candidate humanized antibody functional fragments may also be determined on the basis of the plurality of selected candidate heavy chain variable region sequences and/or the plurality of selected candidate light chain variable region sequences. Here, a humanized antibody functional fragment may refer to a fragment which retains at least a part of the basic function of the initial antibody after humanization processing and which may be a part of the sequence of an intact antibody. For example, a humanized antibody functional fragment may comprise an scFv fragment (single-chain variable fragment), a Fab fragment (antigen-binding fragment), sdAb (single domain antibody), etc. An scFv fragment is an antibody fragment formed from a heavy chain variable region and a light chain variable region of an antibody that are linked via a short peptide (linker) of 15-20 amino acids, and the antibody fragment has characteristics such as antigen-binding property, strong penetrability, a short half-life *in vivo,* low immunogenicity, the ability to be expressed in a prokaryotic cell system, and ease of genetic engineering operation. A Fab fragment consists of an intact light chain (a variable region and a constant region) and a part of a heavy chain structure (a variable region and one constant region fragment) that are linked via a disulfide bond. The Fab fragment has both an antigen binding region and a part of a constant region, which allows the Fab fragment not only to have the same antibody-antigen affinity as the scFv fragment, excellent tissue penetration, etc., but also to have a more stable structure, thereby playing a huge role in clinical diagnosis and treatment. sdAb is an antibody fragment comprising only a single variable domain of the entire antibody, and has high thermal stability and tolerance to denaturants (urea), proteases and low pH environments of the digestive tract, is more soluble in water, has high tissue penetration that enables the crossing of the blood-brain barrier, can recognize small epitopes deep in the molecule that cannot be bound by other forms of antibodies, and thus has great potential for biochemical research and development of new diagnostic and therapeutic methods.

In some embodiments, the candidate humanized antibody sequence may be a monoclonal antibody sequence, a bispecific antibody sequence or a multispecific antibody sequence. In some embodiments, candidate variable region sequences of two or more different initial antibodies may be determined on the basis of steps 110-140 described above or similar manners. On the basis of the candidate heavy variable regions and light chain variable regions of the two or more different initial antibodies, bispecific antibody sequences or multispecific antibody sequences can be further designed. Bispecific/multispecific antibodies have two or more specific antigen binding sites simultaneously, and have characteristics of stronger targeting property and lower toxicity compared with traditional monoclonal antibodies.

In step 160, one or more target humanized antibody sequences and/or target humanized antibody functional fragment sequences may be determined from the plurality of candidate humanized antibody sequences.

In some embodiments, further screening may be performed against a plurality of candidate humanized antibody sequences or a plurality of candidate humanized antibody functional fragment sequences obtained from the combination in step 150. For example, by means of solubility prediction, post-translational modification site prediction, antigen-antibody molecule docking evaluation, protein stability evaluation, etc., performed by the computing device 600, the superior candidate humanized antibody sequences that meet the requirements can be selected. For more details on screening candidate humanized antibody sequences, see FIG. 5 and the description thereof.

In some embodiments, corresponding candidate humanized antibody molecules may be synthesized, according to the selected candidate humanized antibody sequences, for actual experimental detection. The user may determine candidate humanized antibody molecules with good performance (e.g., strong affinity for antigen, high stability, and high specificity) as target humanized antibody molecules and conduct further research. The user may also input the selected target humanized antibody molecules into the computing device for documentation and subsequent analysis. By means of simulation and prediction and evaluation by the computing device described above, candidate variable region sequences and candidate humanized antibody sequences are screened separately, and the obtained candidate humanized antibody sequences have better properties in various aspects, which can save the costs and the time and efforts consumed in the antibody molecule synthesis stage and the experimental detection stage. In some embodiments, after synthesizing humanized antibody functional fragments according to the selected candidate humanized antibody functional fragment sequences, the experimental detection may also be performed in a similar manner to determine the target humanized antibody functional fragments.

In some embodiments, if the candidate humanized antibody molecules or candidate humanized antibody functional fragments do not meet the requirements, the computing device 600 may reselect the target variable region sequence template and perform an iterative simulated evolution again using the genetic algorithm to obtain new candidate variable region sequences, thereby obtaining new candidate humanized antibody molecules and/or candidate humanized antibody functional fragments. For example, the computing device may select a human-derived variable region sequence that has the second highest similarity to the variable region sequence of an antibody from the database containing a plurality of human-derived variable region sequences as a target variable region sequence template. In some embodiments, if the candidate humanized antibody molecules or the candidate humanized antibody functional fragments do not meet the requirements, the user may adjust genetic algorithm-related parameters, e.g., adjust parameters such as the probability of mutation and the probability of migration. In some embodiments, the user may also adjust the termination condition of the genetic algorithm, e.g., increase the total number of iterative simulated evolutions, to obtain more candidate humanized antibody molecules and/or candidate humanized antibody functional fragments.

FIG. 2 is a method for obtaining a plurality of candidate variable region sequences using a genetic algorithm shown according to some embodiments of the present description. In some embodiments, a process 200 describes an exemplary process on the basis of a single-population genetic algorithm, comprising at least step 210 to step 280. In some embodiments, steps 210-280 may be executed on a computing device, such as a computing device 600.

In step 210, a simulated mutation may be executed on a framework region of the first variable region sequence template according to a preset mutation probability on the basis of a first variable region sequence template corresponding to a first variable region in a target variable region sequence template to obtain a plurality of initial populations corresponding to the first variable region, wherein each of the plurality of initial populations comprises a plurality of initial variable region sequences.

In some embodiments, the first variable region refers to a heavy chain variable region or a light chain variable region. Steps 210-280 may be performed on the basis of the heavy chain variable region sequence template in the target variable region sequence template to obtain a plurality of candidate heavy chain variable region sequence templates. Steps 210-280 may also be performed on the basis of the light chain variable region sequence template in the target variable region sequence template to obtain a plurality of candidate light chain variable region templates. For example, the plurality of initial populations may be represented as A₁, A₂, ..., A_{N1}. N1 is the number of initial populations. Each initial population may have N2, e.g., 100, 200, 500, etc., initial variable region sequences.

In some embodiments, a simulated mutation may be performed on each amino acid on the framework region of the first variable region sequence template according to a preset mutation probability M1, and the result may be that: the amino acid is not mutated, mutated to another amino acid or deleted. The deleted amino acid may be represented by the mask X. The preset mutation probability M1 may be 0.01, 0.02, 0.05, etc.

In step 220, a plurality of initial variable region sequences may be selected from the plurality of initial variable region sequences as first sequences on the basis of an individual fitness of each of the plurality of initial variable region sequences.

In some embodiments, for each initial variable region sequence, a trained depth forest model may be used for predicting a humanization probability for the initial variable region sequence, and calculating a distance between the initial variable region sequence and the first variable region sequence template on the basis of an edit distance or on the basis of a substitution scoring matrix of protein sequence alignment. In some embodiments, an NSGA-II or a similar genetic algorithm may be used for determining an individual fitness of the initial variable region sequence on the basis of the humanization probability of the initial variable region sequence and the distance between the initial variable region sequence and the first variable region sequence template.

In step 230, the first sequences of the plurality of initial populations may be retained, and unselected initial variable region sequences of the plurality of initial populations may be removed to obtain a plurality of first populations.

For example, an initial population A₁ comprises an individual a, an individual b, an individual c, etc. An initial population A₂ comprises an individual d, an individual e, an individual f, etc. The individual a, the individual b, the individual d and the individual e are the selected initial variable region sequences, while the individual c and the individual f are the unselected initial variable region sequences, such that a first population A₁' comprises the individual a and the individual b, and a first group A₂' comprises the individual d and the individual e.

In step 240, a simulated migration may be executed between the plurality of first populations to obtain a plurality of second populations, each of the plurality of second populations comprising second sequences.

In some embodiments, a simulated migration between two populations means that each individual in one population has a certain probability (based on the migration rate) of migrating to another population. For example, the individual a in the first population A₁' may be transferred to the first population A₂', such that a second population A₁" comprises the individual b, while a second population A₂" comprises the individual a, the individual d and the individual e.

In step 250, a simulated crossover and a simulated variation may be executed on the second sequences of the plurality of second populations to obtain a plurality of third populations, each of the plurality of third populations comprising a plurality of third sequences.

In some embodiments, the simulated crossover may be a simulation of parent sequences undergoing crossover combinations according to a certain probability (i.e., crossover rate), in which parts of framework regions of the two parent sequences are combined to generate progeny sequences. CDR regions remain unchanged. For example, the framework region of the individual a in the second population A₂" comprises FR1 and FR2, and the framework region of the individual b comprises FR3 and FR4, such that after the simulated crossover occurs between the individual a and the individual b, the framework region of the newly generated individual may comprise FR1+FR3, or FR1+FR4, FR2+FR3 or FR2+FR4. In some embodiments, the crossover rate may be 0.7, 0.8, etc.

In some embodiments, before or after executing a simulated intersection, a simulated variation may also be performed on the framework regions in the sequences according to a preset mutation probability M. The preset mutation probability M in this step and the preset mutation probability M1 in step 210 may be the same or different.

In step 260, one or more candidate variable region sequences may be determined from the plurality of third sequences.

In some embodiments, a manner similar to step 220 may be adopted to determine an individual fitness of each third sequence, and select one or more candidate variable region sequences from the third sequences on the basis of the individual fitness.

In step 270, whether a termination condition is reached may be judged. If the termination condition is not reached, go back to step 220 and repeat steps 220-270. If the termination condition is reached, step 280 may be executed.

In step 280, a plurality of candidate variable region sequences corresponding to the first variable region may be obtained.

In some embodiments, the candidate variable region sequences determined in each iterative loop may be retained. These retained candidate variable region sequences may be further screened when the termination condition is reached, and combined with constant regions to form intact candidate humanized antibody sequences.

FIG. 3 is a method for obtaining a plurality of candidate variable region sequences using a genetic algorithm shown according to some embodiments of the present description. In some embodiments, a process 300 describes an exemplary process on the basis of a multi-population genetic algorithm, comprising at least step 310 to step 380. In some embodiments, steps 310-380 may be executed on a computing device, such as a computing device 600.

In step 310, a simulated mutation may be executed on a framework region of a first template according to a preset mutation probability on the basis of the first template to obtain a plurality of first initial populations, wherein each of the plurality of first initial populations comprises a plurality of initial variable region sequences.

In step 320, a plurality of initial variable region sequences may be selected from the plurality of initial variable region sequences as first sequences on the basis of an individual fitness of each of the plurality of initial variable region sequences.

In step 330, the first sequences of the plurality of initial populations may be retained, and unselected initial variable region sequences of the plurality of initial populations may be removed to obtain a plurality of first retained populations.

In step 315, a simulated mutation may be executed on a framework region of a second template according to a preset mutation probability on the basis of the second template to obtain a plurality of second initial populations, wherein each of the plurality of second initial populations comprises a plurality of initial variable region sequences.

In some embodiments, the second template is different from the first template. For example, the first template is a human-derived variable region sequence which is determined from the database containing a plurality of human-derived variable region sequences and has the highest similarity to a sequence of a framework region of a first variable region of the initial antibody, and the second template is a human-derived variable region sequence which is determined from the database containing a plurality of human-derived variable region sequences and has the highest similarity to a complete sequence of a first variable region of the initial antibody.

In step 325, a plurality of initial variable region sequences may be selected from the plurality of initial variable region sequences as first sequences on the basis of an individual fitness of each of the plurality of initial variable region sequences.

In step 335, the first sequences of the plurality of initial populations may be retained, and unselected initial variable region sequences of the plurality of initial populations may be removed to obtain a plurality of second retained populations.

In step 340, a simulated migration may be executed among the plurality of first retained populations, among the plurality of second retained populations, and between the first retained populations and the second retained populations to obtain first retained populations and second retained populations after the migration.

In the course of iterative evolution, an individual migration with a certain probability may occur between the two types of populations and within each type of population in each generation. For example, the individual migration probability among the same type of populations is X1, and the individual migration probability among different types of populations is X2. In some embodiments, since the first retained population is obtained on the basis of the first template and the second retained population is obtained on the basis of the second template, the first retained population and the second retained population may be considered to be of different types of populations. The migration may be executed among the same type of populations, e.g., among a plurality of first retained populations, or among a plurality of second retained populations according to the migration rate X1. The migration may be executed among different types of populations according to the migration rate X2. For example, an individual in the first retained population has a probability of X2 to migrate into the second retained population. For the first retained population and the second retained population after the migration, step 350 and step 355 may be executed, respectively, to generate progeny individuals. In some embodiments, X1 and X2 may be the same or different.

In step 350, a simulated crossover and a simulated variation may be executed on the second sequences of the first retained population after the migration to obtain a plurality of third populations, each of the plurality of third populations comprising a plurality of third sequences.

In step 355, a simulated crossover and a simulated variation may be executed on the second sequences of the first retained population after the migration to obtain a plurality of third populations, each of the plurality of third populations comprising a plurality of third sequences.

The population evolutionary crossover rate for executing the simulated crossover in step 350 and step 355 is C, and the population mutation rate for executing the simulated variation in step 350 and step 355 is M. The population evolutionary crossover rate C may be 0.5, 0.6, 0.7, 0.8, 0.9, etc. The population mutation rate M may be 0.001, 0.01, 0.02, 0.03, 0.04, 0.05, 0.06, 0.07, 0.08, etc.

In step 360, one or more candidate variable region sequences may be determined from the plurality of third sequences.

In step 370, whether a termination condition is reached may be judged. If the termination condition is not reached, go back to step 320 and step 325 and repeat steps 320-370 and steps 325-360. If the termination condition is reached, step 380 may be executed.

In step 380, a plurality of candidate variable region sequences corresponding to the first variable region may be obtained.

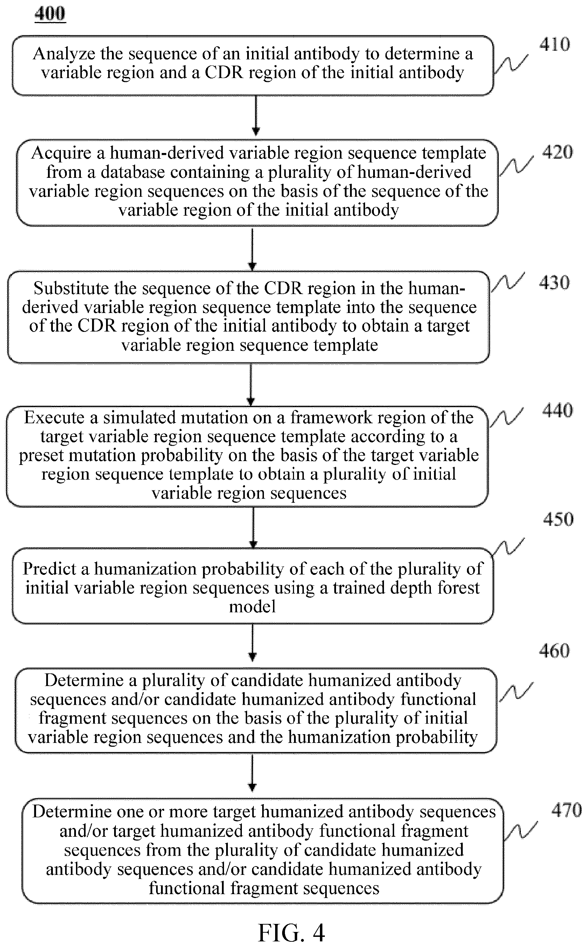

FIG. 4 is a method for designing a humanized antibody sequence shown according to some embodiments of the present description. In some embodiments, a process 400 may be used for designing, on the basis of the initial antibody, a plurality of target antibody sequences and/or a plurality of target antibody functional fragment sequences after humanization. In some embodiments, at least a part of steps 410-470 may be executed on a computing device, such as a computing device 600.

In step 410, sequence analysis may be performed on a sequence of an initial antibody to determine a variable region and a CDR region in the sequence of the antibody.

In step 420, a human-derived variable region sequence template may be obtained from a database containing a plurality of human-derived variable region sequences on the basis of a sequence of the variable region of the initial antibody.

In step 430, a sequence of a CDR region in the human-derived variable region sequence template may be substituted with a sequence of the CDR region of the initial antibody to obtain a target variable region sequence template. In some embodiments, steps 410-430 may be implemented in a similar manner to steps 110-130 in FIG. 1.

In step 440, a simulated mutation may be executed on a framework region of the target variable region sequence template according to a preset mutation probability on the basis of the target variable region sequence template to obtain a plurality of initial variable region sequences. For more descriptions of the simulated mutation and the target variable region sequence template, see elsewhere in the description, such as step 140 in FIG. 1 and step 210 in FIG. 2 and descriptions thereof. As used herein, the term "framework region", when used for describing a part of an antibody variable region, refers to a region of the variable region other than the CDR regions.

In step 450, a humanization probability of each of the plurality of initial variable region sequences may be predicted using a trained depth forest model.

In some embodiments, a deep forest model corresponding to a heavy chain variable region may be used for predicting humanization probabilities for initial variable region sequences corresponding to the heavy chain variable region, and a deep forest model corresponding to a light chain variable region may be used for predicting humanization probabilities for initial variable region sequences corresponding to the light chain variable region. For more descriptions of the humanized probability prediction and the trained deep forest models, see elsewhere in the description, such as step 140.

In step 460, a plurality of candidate humanized antibody sequences and/or candidate humanized antibody functional fragment sequences may be determined on the basis of the plurality of initial variable region sequences and the humanization probabilities.

In some embodiments, a heuristic algorithm may be used for determining a plurality of candidate humanized antibody sequences and/or a plurality of candidate humanized antibody functional fragment sequences on the basis of the plurality of initial variable region sequences and the humanization probability. The heuristic algorithm is an intuitively or empirically constructed algorithm that gives a feasible solution for each instance of a combinatorial optimization problem to be solved at an acceptable cost (referring to computational time and space), and the degree of deviation of the feasible solution from the optimal solution generally cannot be predicted. For example, the heuristic algorithm may comprise a genetic algorithm, a particle swarm algorithm, an ant colony algorithm, a simulated annealing method, a list search algorithm, evolutionary programming, evolutionary strategy, neural networks, etc., or derivative algorithms or combinations thereof. As an example, a genetic algorithm may be used for executing an iterative simulated evolution to determine a plurality of candidate variable region sequences on the basis of the plurality of initial variable region sequences and the humanization probability, and determining the plurality of candidate humanized antibody sequences and/or the plurality of candidate humanized antibody functional fragment sequences on the basis of the plurality of candidate variable region sequences. For more description of executing an iterative simulated evolution using a genetic algorithm, see elsewhere in the description, such as step 140 in FIG. 1, and FIG. 2 and FIG. 3, and related descriptions thereof.

In step 470, one or more target humanized antibody sequences and/or target humanized antibody functional fragment sequences may be determined from the plurality of candidate humanized antibody sequences and/or candidate humanized antibody functional fragment sequences. In some embodiments, step 470 may be executed in a manner similar to step 160.

FIG. 5 is a method for determining one or more target humanized antibodies on the basis of a plurality of candidate variable region sequences shown according to some embodiments of the present description. In some embodiments, a process 500 may be used for further screening the candidate variable region sequences and further screening the candidate humanized antibody sequences, thereby obtaining target humanized antibody sequences. In some embodiments, the process 500 comprises at least steps 510-570. In some embodiments, step 520, step 550 and step 560 are optional. In some embodiments, at least a part of steps 510-570, such as steps 510-560, may be executed on a computing device, such as a computing device 600.

In step 510, a variable region sequence database may be obtained.

**The** variable region sequence database may comprise various human-derived and non-human-derived variable region sequences. For example, the variable region sequence database may comprise a plurality of sample variable region sequences that are derived from human and non-human species and are used for training deep forest models for outputting humanization probabilities. For another example, an NCBI protein non-redundant database may also be acquired and combined with sample variable region sequences to obtain the variable region sequence database.

In step 520, a back mutation or a germ cell lineage antibody gene mutations may be performed on the basis of a plurality of candidate variable region sequences, wherein the plurality of candidate variable region sequences comprises a plurality of candidate light chain variable regions and a plurality of candidate heavy chain variable regions.

In some embodiments, the site at which the back mutation or the germ cell lineage antibody gene mutation is performed may be determined empirically or according to a mutation site prediction method. For example, according to the reported important sites of antibodies from various species, the candidate variable region sequences output using the genetic algorithm may be subjected to back mutations on the basis of non-human-derived antibody sequences or germ cell lineage antibody gene (i.e., germline) mutations of non-human species for light and heavy chains. For example, after a rabbit-derived antibody is encoded by Kabat, back mutations may be considered at positions 49, 71-78 and 94 of the heavy chain and positions 1, 2 and 66-71 of the light chain.

In step 530, for each of the plurality of candidate variable region sequences, a human derivation review may be performed on the basis of the variable region sequence database, and candidate variable region sequences that do not meet the requirements may be filtered according to the review results.

In some embodiments, using a sequence alignment tool (e.g., Blastp), the variable region sequences finally obtained using the genetic algorithm may be combined with the variable region sequences obtained by the back mutations or germ cell lineage antibody gene mutations in step 420, and then compared with the variable region sequence database. If the sequence having the highest overall score (e.g., the total score value in the Blastp output) found by the query sequence is a human-derived antibody sequence, the candidate variable region sequence is retained. If the sequence having the highest overall score is not a human-derived antibody sequence, the candidate variable region sequence is considered not to meet the requirements, and the candidate variable region sequence is not retained.

In step 540, the plurality of candidate light chain variable regions and the plurality of candidate heavy chain variable regions can be combined pairwise and linked to constant regions to determine a plurality of first candidate humanized antibody sequences.

In some embodiments, a human-derived heavy chain constant region (such as the designed or screened heavy chain constant region of IgG1) and a human-derived light chain constant region (such as the designed or screened light chain constant region of IgG1) may be added to the heavy chain variable region and the light chain variable region filtered by means of a humanization review, respectively, and a set of full-length heavy chain sequences and a set of full-length light chain sequences may be arbitrarily and fully combined pairwise to form an antibody dataset.

In step 550, for each of the plurality of candidate humanized antibody sequences, a solubility prediction and a post-translational modification site prediction may be performed, and a plurality of second candidate humanized antibody sequences may be determined from the plurality of first candidate humanized antibodies on the basis of the prediction results.

In some embodiments, software such as DeepSCM or DeepSol may be used for solubility prediction. Software such as MusiteDeep may be used for post-translational modification (PTM) site prediction. This is not limited by the present invention. According to the prediction results, combinations of light and heavy chains that do not meet the preset criteria (such as those containing amino acid sites with PTMs that need to be avoided and those having too low solubility prediction values) may be deleted to determine a plurality of second candidate humanized antibody sequences that meet the preset criteria.

In step 560, an antigen-antibody molecule docking evaluation and a protein stability evaluation may be performed on the plurality of second candidate humanized antibodies, and according to the evaluation results, one or more third candidate humanized antibody sequences are determined from the plurality of second candidate humanized antibodies.

In some embodiments, protein structure simulation may be performed using antibody prediction software such as IgFold or DeepAb, antigen structure may be determined according to structural biological experimental results or predicted using protein prediction software such as AlphaFold2, molecular docking of antibodies and antigens is performed using ZDOCK, DLAB or other software, and antibodies with top-ranked docking scores are selected. For the protein stability detection, computational evaluation and preliminary screening may also be performed first by means of molecular dynamics simulation of force fields.

In step 570, the one or more third candidate humanized antibodies may be prepared, the performance of the one or more third candidate humanized antibodies may be evaluated experimentally, and according to the performance evaluation results, one or more target humanized antibodies may be determined from the one or more third candidate humanized antibodies.

In some embodiments, experiments such as an expression test, an antigen antibody affinity test and a stability test may be performed on the synthesized third candidate humanized antibodies to evaluate the performance of the third candidate humanized antibodies and determine the target humanized antibodies from the third candidate humanized antibodies. In some embodiments, step 520, step 550 and step 560 are optional. The candidate antibodies with top-ranked homology may also be selected directly from the first candidate humanized antibody sequences determined in step 540 for downstream experimental validation.

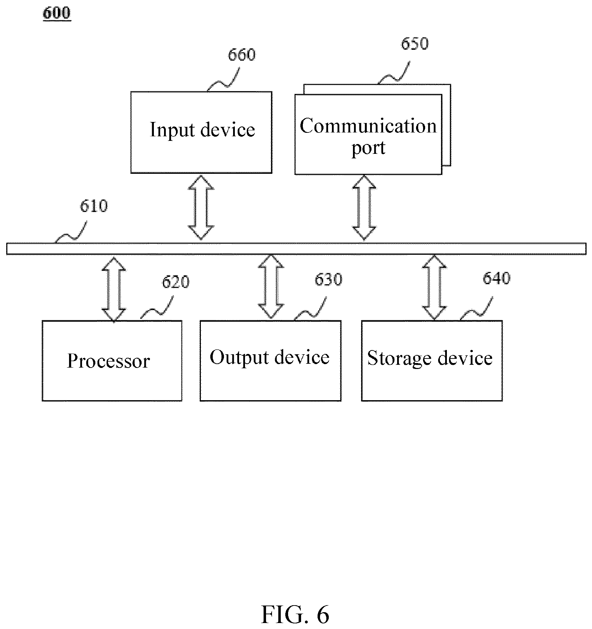

FIG. 6 is a schematic diagram of an exemplary computing device shown according to some embodiments of the present description. The computing device 600 may be a general-purpose computer or a special-purpose computer. The computing device 600 may be used for executing the method for designing a humanized antibody sequence as described herein. In FIG. 6, for convenience, only one such computing device is shown. It will be understood by those of ordinary skill in the art that the computing device 600 may be implemented in a distributed manner on a plurality of similar platforms to distribute processing loads.

For example, the computing device 600 may comprise an internal communication bus 610 and a communication port 650 connected with a network to facilitate data communication. The computing device 600 may also comprise a processor 620 in the form of one or more processors used for executing program instructions. The computing device 600 may further comprise storage device 640 used for storing various forms of data and instructions. For example, the instructions may be run in processor 620 to execute the steps in the method for designing a humanized antibody sequence provided in some embodiments of the description (such as steps 110-160 in the process 100 or steps 410-470 in the process 400). The storage device 640 may comprise various storage components such as disk and read-only memory (ROM) random access memory (RAM). The computing device 600 may further comprise an output device 630 and an input device 660 that are used for supporting input/output between the computing device 600 and other devices. The computing device 600 may also receive data from external devices via a communication network.

For illustration only, only one processor 620 is described in the computing device 600. However, it should be noted that the computing device 600 in the present application may further comprise a plurality of processors. Therefore, operations and/or method steps executed by one processor as described in the present application may also be executed jointly or separately by a plurality of processors. For example, in the present application, if the processor of the computing device 600 executes an operation A and an operation B, it should be understood that the operation A and the operation B may also be executed jointly or separately by two or more different processors in the computing device 600 (e.g., a first processor executes the operation A and a second processor executes the operation B, or the first and second processors perform the operations A and B jointly).

**Yet** another aspect of the present description further provides a non-transitory computer-readable storage medium having stored thereon an instruction set used for designing a humanized antibody sequence. When the instruction set is executed by a computing device, such as the computing device 600, causes the computing device to execute the method for designing a humanized antibody as described herein, such as steps 110-160 in the process 100 or steps 410-470 in the process 400.

Beneficial effects that may result from the method for designing a humanized antibody sequence disclosed in the present description comprise, but are not limited to: (1) In the embodiments of the present description, by performing an iterative simulated evolution using a genetic algorithm on the basis of a variable region sequence template, by screening for optimized mutated individual sequences on the basis of humanization probabilities and distances between the individual sequences and the variable region sequence template, and by performing a simulated migration, a simulated crossover and further individual mutation, a large number of optimized candidate variable region sequences can be efficiently generated, saving time and effort in designing and screening humanized antibody sequences; moreover, by the iterative optimization process described above, the performance of the obtained humanized antibody sequences, such as the affinity for an antigen, can be improved; (2) In the embodiments of the present description, by predicting the humanization probabilities of the sequences by means of a deep forest model, data of different sizes can be processed; the deep forest model has more stable and better learning performance, is not demanding on data size, features a rapid training speed, and can reduce computational and time costs; compared with other deep learning models (e.g., deep neural networks), there is no need to set complex hyper-parameters, and the corresponding parameters are automatically set by minimizing the training error on specific datasets, that is, the modeling difficulty is reduced; in addition, the tree structure of the deep forest has better interpretability than neural networks, which is conducive to mining and interpreting deep biological meanings. It should be noted that different embodiments may have different beneficial effects, and the beneficial effects that may be produced in the different embodiments may be any one or a combination of the above, or may be any other beneficial effects that may be obtained.

The experimental methods in the following examples are all conventional methods, unless otherwise specified. The experimental materials used in the following examples were all purchased from conventional biochemical reagent companies, unless otherwise specified. The quantitative experiments in the following examples were performed in triplicate, and the results were averaged.

### Example 1 Determination of variable region sequences and CDR region sequences of anti-CD137 rabbit antibody

Rabbit monoclonal antibodies have higher affinity, higher specificity and higher sensitivity, and are more homologous to human antibodies compared with murine antibodies, can even recognize subtle antigen changes (such as methylation and phosphorylation), and thus are the most promising platform for antibody drug development. In the present invention, the humanization of rabbit antibodies is taken as an exemplary example to illustrate the process of using the method for designing a humanized antibody sequence provided by the present invention.
(1) Acquisition of the original sequence of a rabbit antibody.

TNFRSF9 protein (also referred to as CD137 protein), with the Accession number NP_001552.2 in the NCBI database, was selected as an antigen, and the antigen sequence was: MGNSCYNIVATLLLVLNFERTRSLQDPCSN CPAGTFCDNNRNQICSPCPPNSFSSAGGQRTCDICRQCKGVFRTRKECSSTSNAECDCT PGFHCLGAGCSMCEQDCKQGQELTKKGCKDCCFGTFNDQKRGICRPWTNCSLDGKSVL VNGTKERDVVCGPSPADLSPGASSVTPPAPAREPGHSPQIISFFLALTSTALLFLLFFLTLR FSVVKRGRKKLLYIFKQPFMRPVQTTQEEDGCSCRFPEEEEGGCEL (SEQ ID NO: 1).

The MonoRab^{™} rabbit monoclonal antibody customization service of Nanjing Genscript Biotech Co., Ltd. (https://www.genscript.com.cn/custom-rabbit-monoclonal-antibody-generation.html) was ordered to prepare a rabbit monoclonal antibody against the antigen.

**(2)** Variable region sequence determination and CDR annotation of the original antibody.

After sequence determination, the heavy chain variable region sequence of the rabbit antibody obtained in step (1) was: QSVEESGGRLVTPGTPLTLTC TVSGFSLGTFPIGWVRQAPGSGLEWIGIFNTDGSAAYASWARGRFTVFKNSTTVDLKMTS PTTEDTATYFCARINADYSGWYFGIWGPGTLVTVSS (SEQ ID NO: 2). After IMGT numbering, on the basis of North definition, the heavy chain CDR1, 2 and 3 of the antibody were TVSGFSLGTFPIG (SEQ ID NO: 3), IFNTDGSAA (SEQ ID NO: 4) and ARINADYSGWYFGI (SEQ ID NO: 5), respectively.

After sequence determination, the light chain variable region sequence of the rabbit antibody obtained in step (1) was: EMTQTPASVEVAVGGTVTIKCQ ASQSIYSYLAWYQQKPGQPPKPLIYEASKTPSGVPSRFKGSGSGTEYTLTISGVQCEDAA TYYCQKGYSVSNVAFGGGTEVVVK (SEQ ID NO: 6). After IMGT numbering, on the basis of North definition, the light chain CDR1, 2 and 3 of the antibody were QASQSIYSYLA (SEQ ID NO: 7), YEASKTPS (SEQ ID NO: 8) and QKGYSVSNVA (SEQ ID NO: 9), respectively.

(3) Length standardization processing of antibody variable regions.

**The** antibody heavy chain variable region was subjected to length standardization to obtain qH_formatted, which was QXSVEESGGXRLVTPGT PLTLTCTVSGFSLXXXXGTFPIGWVRQAPGSGLEWIGIFNTDXXXGSAAYASWARXGRFT VFKNSTXXTVDLKMTSPTTEDTATYFCARINADYXXXXXXXXXXXXXXXXXXXXXXXSGW YFGIWGPGTLVTVSS (SEQ ID NO: 10).

**The** antibody light chain variable region was subjected to length standardization to obtain qKL_formatted, which was XXEMTQTPASVEVA VGGTVTIKCQASQSIXXXXXXYSYLAWYQQKPGQPPKPLIYEAXXXXXXXSKTPSGVPXS RFKGSGXXSGTEYTLTISGVQCEDAATYYCQKGYSXXXXXXXXXXVSNVAFGGGTEVVV K (SEQ ID NO: 11).

### Example 2 Determination of target variable region sequence templates of anti-CD137 rabbit antibody

For the qH_formatted obtained in step (3) of Example 1, a human-derived template tmp_Hchain-fullSeq was found after the blastp alignment. The sequence thereof was:

EVQLVESGGXGLVQPGRSLRLSCTASGFTFXXXXGDYAMSWV
RQAPGRGLEWIGFIRSKVYDGTTEYAASVKXGRFTISRDDSKSIAHLQMNSLTTEDTAVYY
CSRLRADSSDYYXXXXXXXXXXXXXXXXXSSPGSYYFDYWGQGTLVTVSS (SEQ ID NO:
12).

CDR grafting was performed on tmp_Hchain-fullSeq using heavy chain CDR1, 2 and 3 of the rabbit antibody to generate corresponding template_Hchain-fullSeq, which, after removing masks "X", was EVQLVESGGGLVQPGR SLRLSCTVSGFSLGTFPIGVWRQAPGRGLEWIGIFNTDGSAAYAASVKGRFTISRDDSKSI AHLQMNSLTTEDTAVYYCARINADYSGWYFGIWGQGTLVTVSS (SEQ ID NO: 13).

Alternatively, CDRs of qH_formatted may be first masked with "X" masks to obtain a sequence QXSVEESGGXRLVTPGTPLTLTCXXXXXXXXX XXXXXXXXWVRQAPGSGLEWIGXXXXXXXXXXXXYASWARXGRFTVFKNSTXXTVDLKM TSPTTEDTATYFCXXXXXXXXXXXXXXXXXXXXXXXXXXXXXXXXXXXXXWGPGTLVTVS S (SEQ ID NO: 14), and the sequence of a human-derived template tmp_Hchain-FR was then found after the blastp alignment:

EVQLEESGGXGLVQPGGSLKLSCAASGFTLXXXXSGSSMHWV
RQAPGKGLEWVGRIRSKTNSNATAYAASVKXGRFTVSRDDSKNTVYLHMNSLKTEDTAV
YFCTRTMTLXXXXXXXXXXXXXXXXXXXXXXXXXXXXAAFDYWGQGTLVTVSS (SEQ ID
NO: 15).

CDR grafting was performed on tmp_Hchain-FR using heavy chain CDR1, 2 and 3 of the rabbit antibody to generate corresponding template_Hchain-FR, which, after removing masks "X", was EVQLEESGGGLVQPGGSLKLS CTVSGFSLGTFPIGWVRQAPGKGLEWVGIFNTDGSAAYAASVKGRFTVSRDDSKNTVYL HMNSLKTEDTAVYFCARINADYSGWYFGIWGQGTLVTVSS (SEQ ID NO: 16).

For the qKL_formatted obtained in step (3) of Example 1, a human-derived template tmp_KLchain-fullSeq was found after the blastp alignment, and the sequence thereof was:

DIQMTQSPSSLSVSVGGRVTITCRASQSIXXXXXXSSYLNWYQ
QKPGKAPKLLIYAAXXXXXXXSSLQSGVPXSRFNGNGXXSGTDFTLTISSLQPEDSATYYC
QQSYXXXXXXXXXXXXXSILLFGGGTEVEIK (SEQ ID NO: 17).

CDR grafting was performed on tmp_KLchain-fullSeq using light chain CDR1, 2 and 3 of the rabbit antibody to generate corresponding template_KLchain-fullSeq, which, after removing masks "X", was DIQMTQSPSSLSVSVG GRVTITCQASQSIYSYLAWYQQKPGKAPKLLIYEASKTPSGVPSRFNGNGSGTDFTLTISS LQPEDSATYYCQKGYSVSNVAFGGGTEVEIK (SEQ ID NO: 18).

Alternatively, CDRs of qKL_formatted may be first masked with "X" masks to obtain a sequence XXEMTQTPASVEVAVGGTVTIKCXXXXXXXX XXXXXXXXXWYQQKPGQPPKPLIXXXXXXXXXXXXXXXGVPXSRFKGSGXXSGTEYTLTI SGVQCEDAATYYCXXXXXXXXXXXXXXXXXXXXFGGGTEVVVK (SEQ ID NO: 19), and a human-derived template tmp_KLchain-FR was then found after the blastp alignment. In this example, the sequence of the human-derived template tmp_KLchain-FR was the same as that of tmp_KLchain-fullSeq, and was also DIQMTQSPSSLSVSVGGRVTITCRASQSIXXXXXXSSYLNWY QQKPGKAPKLLIYAAXXXXXXXSSLQSGVPXSRFNGNGXXSGTDFTLTISSLQPEDSATYY CQQSYXXXXXXXXXXXXSILLFGGGTEVEIK (SEQ ID NO: 17), so after performing CDR grafting on tmp_Hchain-FR with light chain CDR1, 2 and 3 of the rabbit antibody, the generated corresponding template_KLchain-FR was also the same as template_Hchain-fullSeq, and was also DIQMTQSPSSL SVSVGGRVTITCQASQSIYSYLAWYQQKPGKAPKLLIYEASKTPSGVPSRFNGNGSGTDF TLTISSLQPEDSATYYCQKGYSVSNVAFGGGTEVEIK (SEQ ID NO: 18) after removing the masks "X".

### Example 3 Genetic algorithm optimization and back mutation based on deep forest

Using the deep-forest package in Python (https://github.com/kingfengji/gcForest) and default parameters thereof, heavy and light chain variable region sequence samples of human-derived and non-human-derived antibodies acquired from the database were subjected to length standardization processing to obtain data of a length-standardized heavy chain group and a length-standardized light chain group (H_chain_strandard contained 129,838,794 sequences, and KL_chain_strandard contained 8,165,055 sequences), and deep forest classification models DFmodel-H and DFmodel-KL were obtained by training, respectively. When using the models, for a heavy chain variable region of an antibody of any species origin, after being judged by DFmodel-H, a probability pH of being classified as human-derived will be output; for a light chain variable region of an antibody of any species origin, after being judged by DFmodel-KL, a probability pKL of being classified as human-derived will be output.

Using the depth forest model DFmodel-H (corresponding to the heavy chain variable region), the humanization probabilities of template_Hchain-fullSeq and template_Hchain-FR were predicted to be 0.695 and 0.4525, respectively. Using the deep forest model DFmodel-KL (corresponding to the light chain variable region), the humanization probabilities of template_KLchain-fullSeq and template_KLchain-FR were predicted. Since the sequences of the two were the same, their probabilities of being human-derived were both 0.9925. On the basis of a multi-population genetic algorithm of NSGA-II, the variable region sequence templates were subjected to a simulated evolution to obtain a plurality of candidate variable region sequences (performed with reference to the process 300, wherein, the number of populations on the basis of template_Hchain-FR (heavy chain first template), template_Hchain-fullSeq (heavy chain second template, template_KLchain-FR (light chain first template) and template_KLchain-fullSeq (light chain second template) were all 3, the sequence of each individual in the initial population was randomly generated by mutating the positions other than CDRs to other amino acids or mask "X" according to a preset mutation probability of 0.05, and the number of individuals in each group was 100. The crossover rate C at which crossovers can occur among individuals in the population was 0.8, the mutation rate for all individuals was 0.05, the migration rates X1 and X2 were both 0.01, and the termination condition was the completion of 1000 optimization generations). In this implementation, the heavy chain variable region sequence having the highest humanization probability (0.8325) and the light chain variable region sequence having the highest humanization probability (1) were selected for subsequent steps. The output heavy and light chain variable region sequences were subjected to back mutations to varying degrees, with the back mutation sites covering positions 49, 71-78 and 94 of the heavy chain and positions 1, 2 and 66-71 of the light chain according to the Kabat numbering.

### Example 4 Judgment of human derivation after humanization

**The** heavy chain variable region and light chain variable region of the antibody were processed according to the process 500 to acquire optimized humanized sequence combinations, and the following four groups were randomly selected from the sequence combinations of the heavy and light chain variable regions of the antibody after humanization as candidate antibodies for protein expression and affinity test. The human derivation of each antibody was judged using Blastp. Accession numbers in the NCBI database of the homologous sequences with the highest scores found were shown in Table 1 below.

**Table 1. Accession numbers in the NCBI database of the homologous sequences with the highest scores found for the four groups of candidate antibodies**

| Variable region ID of candidate antibodies after humanizatio n | Heavy chain variable region sequence | Information of the most homologous sequence of the heavy chain variable region in the NCBI protein non-redundant database | Light chain variable region sequence | Information of the most homologous sequence of the light chain variable region in the NCBI protein non-redundant database |
|---|---|---|---|---|
| 4C2-Candi1 | EVQLEESGGGL VQPGGSLALSC TVSGFSLGTFPI GWVRQAPGKG LEWVGIFNTDG SAAYAASVKGR FTVSRDSSKNTF YLHMNSLKTED TAVYFCARINAD YSGWYFGIWGQ GTLVDVSS (SEQ ID NO: 20) | Human antibody heavy chain variable region sequence QWO16013.1 | DIQMTQSPSSLS VSVGGRVTITCQ ASQSIYSYLAWY QQKPGKAPKLLI YEASKTPSGVP SRFNGNGSGTD FTLTISSLQPED SATYYCQKGYS VSNVAFGGGTE VEIK (SEQ ID NO: 18) | Human antibody light chain variable region sequence UDP68830.1 |
| 4C2-Candi2 | EVQLEESGGGL VQPGMSLRLSC TVSGFSLGTFPI GWVRQAPGKG LEWVGIFNTDG SAAYAASVKGR | Human antibody heavy chain variable region sequence AXA12146.1 | AQQMTQSPSSL SVSVGGRVTITC QASQSIYSYLA WYQQKPGKAPK LLIYEASKTPSG VPSRFNGNGSG | Human antibody light chain variable region sequence CAB51294.1 |
| | FNVSKNSTKNT VYLHMNSLKTE DTAVYFCARINA DYSGWYFGIWG QGTLVTVSS (SEQ ID NO: 21) | | TEYTLTISSLQP EDSATYYCQKG YSVSNVAFGGG TEVEIK (SEQ ID NO: 24) | |
| 4C2-Candi3 | EVQLEESGGGL VQPGGSLALSC TVSGFSLGTFPI GWVRQAPGKG LEWVGIFNTDG SAAYAASVKGR FTVSRDSSKNT VYLHMNSLKTE DTAVYFCARINA DYSGWYFGIWG QGTLVTVSS (SEQ ID NO: 22) | Human antibody heavy chain variable region sequence QWO16013.1 | DIQMTQSPSSLS ASVGGRVTITCQ ASQSIYSYLAWY QQKPGKAPKLLI YEASKTPSGVP SRFNGNGSGTD FTLTISSLQPED SATYYCQKGYS VSNVAFGGGTE VEIK (SEQ ID NO: 25) | Human antibody light chain variable region sequence CAB51294.1 |
| 4C2-Candi4 | EVQLEESGGGV VQPGGSLKLSC TVSGFSLGTFPI GWVRQAPGKG LEWVGIFNTDG SAAYAASVKGR FTVSKNSTKNTV YLHMNSLKTED TAVYFCARINAD YSGWYFGIWGQ GTLVTVSS (SEQ ID NO: 23) | Human antibody heavy chain variable region sequence AXA12146.1 | DIQMTQSPSSLS VSVGGRVTITCQ ASQSIYSYLAWY QQKPGKAPKLLI YEASKTPSGVP SRFNGNGSGTE YTLTISSLQPED SATYYCQKGYS VSNVAFGGGTE VEIK (SEQ ID NO: 26) | Human antibody light chain variable region sequence CAB51294.1 |

As can be seen from the Blastp results, the closest sequences of the antibody variable regions after humanization were all human-derived antibody sequences.

### Example 5 Affinity detection of rabbit-derived monoclonal antibodies before and after humanized optimization

**The** heavy chain variable region and the light chain variable region of a candidate antibody were processed as follows:
**(i)** a signal peptide MGWSCIILFLVATATGVHS (SEQ ID NO: 29) was added to the N-terminus of each;
(ii) a human IgG1 heavy chain constant region sequence ASTKGPSVFPLAPSSKSTSGGTAALGCLVKDYFPEPVTVSWNSGALTSGVHTFPAVLQSS GLYSLSSVVTVPSSSLGTQTYICNVNHKPSNTKVDKKVEPKSCDKTHTCPPCPAPELLGG PSVFLFPPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQVYTLPPSRDEL TKNQVSLTCLVKGFYPSDIAVEWESNGQPENNYKTTPPVLDSDGSFFLYSKLTVDKSRW QQGNVFSCSVMHEALHNHYTQKSLSLSPGK (SEQ ID NO: 27) was added to the C-terminus of the heavy chain variable region to form an intact heavy chain;
**(iii)** a human light chain constant region sequence 

 (SEQ ID NO: 28)
was added to the C-terminus of the light chain variable region to form an intact light chain. The complete sequence of an antibody was sent to Nanjing Genscript Biotech Co., Ltd. for the total synthesis of the antibody and the detection of antigen-antibody affinity.

**The** expression vector pcDNA3.4 carrying the original variable region sequences of the rabbit antibody and also carrying the human IgG1 constant region (the sequence was the same as above), and the candidate antibodies after humanization were all transiently transfected into CHO-S cells for antibody expression. The supernatant was collected, and the antibody binding activity was verified by the ELISA method described below. The concentrations of the antibodies in the expression supernatant were mostly between 1 µg/ml and 50 µg/ml. A stock solution and a 100-fold dilution were taken for the detection. Generally, the 100X dilution is considered to be strongly positive (i.e., having relatively good affinity for an antigen) when the OD450 value by the ELISA assay is greater than 1.0. Compared with the wild-type antibody (i.e., rabbit monoclonal antibody 4C2-WTH before humanization), the determination results of the OD 450 values of the 4 antibodies after optimization were as shown in Table 2 below:

**Table 2. OD450 values for 100-fold dilution of humanized antibodies by ELISA affinity assay**

| No. | Clone No. | 100-fold dilution |
|---|---|---|
| 11 | 4C2-Candi1 | 0.097 |
| 12 | 4C2-Candi2 | 3.481 |
| 13 | 4C2-Candi3 | 1.983 |
| 14 | 4C2-Candi4 | 3.594 |
| 15 | 4C2-WTH | 3.325 |

Therefore, on the basis of the determination of OD 450 values, we selected three clones with OD 450 values greater than 1.0 (i.e., 4C2-Candi2, 4C2-Candi3 and 4C2-Candi4), and the half maximal effective concentration (i.e., EC50) was determined according to the indirect ELISA method, and used for the evaluation of the binding ability of purified antibodies to an antigen TNFRSF9 protein. The steps were as follows: An ELISA plate was coated with 0.5 µg/ml recombinant TNFRSF9 protein in PBS at 100 µl/well overnight at 4°C. The plate was washed with PBS-T (0.05% Tween) and blocked with PBST containing 1% BSA at 250 µl/well for 2 hours at 37°C. The blocking buffer was then discarded. 100 µl of 1 µg/ml purified antibody was added to the first well, and 3-fold serial dilutions were performed in other wells, with a total of 11 test concentration gradients, and one blank well was set. Then, the plate was incubated for 1 hour at 37°C. The plate was washed three times with PBST and incubated with horseradish peroxidase-conjugated goat anti-mouse IgG (Fc-specific) secondary antibody (Jackson, 115-035-071) at 100 µl/well for 0.5 hours at 37°C. The plate was washed four times with PBST. Then, a TMB chromogenic solution (GenScript) was added, and the plate was incubated for 15 minutes at 25°C in the dark. The reaction was stopped by adding 50 µl of 1 M HCl stop buffer (Sinopharm, 10011018). The plate was read at 450 nm using a plate reader and the EC50 values were shown in Table 3:

**Table 3. EC50 values for affinity of humanized antibodies by ELISA assay**

| Affinity ranking (high to low) | Clone No. | EC50 (ng/ml) |
|---|---|---|
| 1 | 4C2-Candi2 | 2.383 |
| 2 | 4C2-Candi4 | 2.456 |
| 3 | 4C2-WTH | 3.796 |
| 4 | 4C2-Candi3 | 100 |

**The** affinity of 4C2-Candi3 was 1/26.3 of that of 4C2-WTH. However, the EC50 values of 4C2-Candi2 and 4C2-Candi4 after humanization (the smaller the value, the higher the affinity) were even better than that of 4C2-WTH. This suggests that the affinity of the obtained humanized antibody sequences by means of the iterative optimization process of the genetic algorithm is improved compared with the initial rabbit antibody, further demonstrating that the method for designing a humanized antibody sequence provided in the present description can improve the performance of humanized antibodies.

It should be understood by those skilled in the art that the above examples are only for illustrating the present invention and do not constitute any limitation of the present description. Any modifications, equivalent substitutions, changes, and the like made within the spirit and principle of the present invention shall fall within the scope of protection of the present invention.

## Claims

1. A method for designing a humanized antibody sequence, wherein the method comprises:
performing sequence analysis on a sequence of an initial antibody to determine a variable region and a CDR region of the initial antibody;
acquiring a human-derived variable region sequence template from a database containing a plurality of human-derived variable region sequences on the basis of the sequence of the variable region of the initial antibody;
substituting the sequence of the CDR region in the human-derived variable region sequence template into the sequence of the CDR region of the initial antibody to obtain a target variable region sequence template;
executing iterative simulated evolution by means of using a genetic algorithm on the basis of the target variable region sequence template to determine a plurality of candidate variable region sequences;
determining a plurality of candidate humanized antibody sequences and/or a plurality of candidate humanized antibody functional fragment sequences on the basis of the plurality of candidate variable region sequences; and
determining one or more target humanized antibody sequences and/or target humanized antibody functional fragment sequences from the plurality of candidate humanized antibody sequences and/or the plurality of candidate humanized antibody functional fragment sequences.

2. The method according to claim 1, wherein the executing iterative simulated evolution by means of using a genetic algorithm on the basis of the target variable region sequence template to determine a plurality of candidate variable region sequences comprises:
executing a simulated mutation on a framework region of the target variable region sequence template according to a preset mutation probability on the basis of the target variable region sequence template to obtain a plurality of initial variable region sequences;
predicting a humanization probability of each of the plurality of initial variable region sequences using a trained depth forest model; and
executing an iterative simulated evolution using the genetic algorithm on the basis of the plurality of initial variable region sequences and the humanization probability to determine a plurality of candidate variable region sequences.

3. The method according to claim 2, wherein the executing an iterative simulated evolution using the genetic algorithm on the basis of the plurality of initial variable region sequences and the humanization probability to determine a plurality of candidate variable region sequences comprises:
determining, for each of the plurality of initial variable region sequences, a distance between the initial variable region sequence and the target variable region sequence template, and determining an individual fitness of the initial variable region sequence on the basis of the distance between the initial variable region sequence and the target variable region sequence template and the humanization probability of the initial variable region sequence; and
executing an iterative simulated evolution on the basis of the plurality of initial variable region sequences and the individual fitness to determine a plurality of candidate variable region sequences.

4. The method according to claim 3, wherein the individual fitness is determined using an NGSA-II algorithm or a derivative algorithm thereof.

5. The method according to claim 3, wherein the target variable region sequence template comprises a target heavy chain variable region sequence template and a target light chain variable region sequence template, and the plurality of candidate variable region sequences comprises a plurality of candidate heavy chain variable region sequences and a plurality of candidate light chain variable region sequences.

6. The method according to claim 5, wherein the executing a simulated mutation on a framework region of the target variable region sequence template according to a preset mutation probability on the basis of the target variable region sequence template to obtain a plurality of initial variable region sequences comprises:
executing a simulated mutation on a framework region of the first variable region sequence template according to the preset mutation probability on the basis of a first variable region sequence template corresponding to a first variable region in the target variable region sequence template to obtain a plurality of initial populations corresponding to the first variable region, wherein each of the plurality of initial populations comprises the plurality of initial variable region sequences corresponding to the first variable region, and the first variable region is a heavy chain variable region or a light chain variable region.

7. The method according to claim 6, wherein each simulated evolution operation in the iterative simulated evolution comprises:
selecting a plurality of initial variable region sequences from the plurality of initial variable region sequences as a plurality of first sequences on the basis of the individual fitness of each of the plurality of initial variable region sequences corresponding to the first variable region;
retaining the plurality of first sequences of the plurality of initial populations and removing unselected initial variable region sequences of the plurality of initial populations to obtain a plurality of first populations;
executing a simulated migration among the plurality of first populations to obtain a plurality of second populations, each of the plurality of second populations comprising a plurality of second sequences;
executing a simulated crossover and a simulated variation on the plurality of second sequences of the plurality of second populations to obtain a plurality of third populations, each of the plurality of third populations comprising a plurality of third sequences; and
determining one or more first candidate variable region sequences from the plurality of third sequences.

8. The method according to claim 7, wherein a termination condition for the iterative simulated evolution comprises at least one of the following conditions:
a total number of rounds of the iterative simulated evolution being greater than or equal to a first threshold;
a total number of first candidate variable region sequences obtained by the iterative simulated evolution being greater than or equal to a second threshold;
a diversity of the one or more first candidate variable region sequences obtained by a current round of simulated evolution operation being less than or equal to a third threshold; and
an average similarity of first candidate variable region sequences obtained by a current round of simulated evolution operation to first candidate variable region sequences obtained by a previous round of simulated evolution operation being greater than or equal to a fourth threshold.

9. The method according to claim 7, wherein the first variable region sequence template comprises a first template and/or a second template, wherein the first template is a human-derived variable region sequence which is acquired from the database containing a plurality of human-derived variable region sequences and has the highest similarity to a sequence of a framework region of a first variable region of the initial antibody, and the second template is a human-derived variable region sequence which is acquired from the database containing a plurality of human-derived variable region sequences and has the highest similarity to a complete sequence of a first variable region of the initial antibody.

10. The method according to claim 9, wherein
the first variable region sequence template comprises the first template and the second template;
the plurality of initial populations comprises a plurality of first initial populations obtained by executing a simulated mutation on the basis of the first template and a plurality of second initial populations obtained by executing a simulated mutation on the basis of the second template; and
the plurality of first populations comprises a plurality of first retained populations obtained by retaining the first sequences from the first initial populations and a plurality of second retained populations obtained by retaining the first sequences from the second initial populations.

11. The method according to claim 10, wherein the executing a simulated migration among the plurality of first populations to obtain a plurality of second populations comprises:
executing a simulated migration among the plurality of first retained populations, executing a simulated migration among the plurality of second retained populations, and executing a simulated migration between the first retained populations and the second retained populations to obtain the plurality of second populations.

12. The method according to claim 2, wherein the trained deep forest model is obtained by the following steps:
acquiring a plurality of training samples corresponding to the first variable region, wherein each of the plurality of training samples comprises a human-derived or non-human-derived variable region sequence and a tag indicating that the sample variable region sequence is human-derived or non-human-derived, and the sample variable region sequence is subjected to length standardization processing; and
training an initial deep forest model using the plurality of training samples to obtain a trained deep forest model.

13. The method according to claim 5, wherein the determining a plurality of candidate humanized antibody sequences and/or a plurality of candidate humanized antibody functional fragment sequences on the basis of the plurality of candidate variable region sequences comprises:
determining a plurality of selected candidate heavy chain variable region sequences from the plurality of candidate heavy chain variable region sequences;
determining a plurality of selected candidate light chain variable region sequences from the plurality of candidate light chain variable region sequences; and
determining the plurality of candidate humanized antibody sequences and/or the plurality of candidate humanized antibody functional fragment sequences on the basis of the plurality of selected candidate heavy chain variable region sequences and the plurality of selected candidate light chain variable region sequences.

14. The method according to claim 1, wherein the performing sequence analysis on a sequence of an initial antibody to determine a variable region and a CDR region of the initial antibody comprises:
performing sequence analysis on the sequence of the initial antibody to determine a heavy chain variable region, a light chain variable region, a heavy chain CDR region, and a light chain CDR region of the initial antibody;
performing length standardization processing on a sequence of the heavy chain variable region; and
performing length standardization processing on a sequence of the light chain variable region.

15. A method for designing a humanized antibody sequence, wherein the method comprises:
performing sequence analysis on a sequence of an initial antibody to determine a variable region and a CDR region of the initial antibody;
acquiring a human-derived variable region sequence template from a database containing a plurality of human-derived variable region sequences on the basis of the sequence of the variable region of the initial antibody;
substituting the sequence of the CDR region in the human-derived variable region sequence template into the sequence of the CDR region of the initial antibody to obtain a target variable region sequence template;
executing a simulated mutation on a framework region of the target variable region sequence template according to a preset mutation probability on the basis of the target variable region sequence template to obtain a plurality of initial variable region sequences;
predicting a humanization probability of each of the plurality of initial variable region sequences using a trained depth forest model;
determining a plurality of candidate humanized antibody sequences and/or a plurality of candidate humanized antibody functional fragment sequences on the basis of the plurality of initial variable region sequences and the humanization probability; and
determining one or more target humanized antibody sequences and/or target humanized antibody functional fragment sequences from the plurality of candidate humanized antibody sequences or functional fragments thereof.

16. The method according to claim 15, wherein the determining a plurality of candidate humanized antibody sequences and/or a plurality of candidate humanized antibody functional fragment sequences on the basis of the plurality of initial variable region sequences and the humanization probability comprises:
determining a plurality of candidate humanized antibody sequences and/or a plurality of candidate humanized antibody functional fragment sequences using a heuristic algorithm on the basis of the plurality of initial variable region sequences and the humanization probability.

17. The method according to claim 16, wherein the heuristic algorithm comprises at least one of the following algorithms: a genetic algorithm, a particle swarm algorithm, an ant colony algorithm, a simulated annealing method, a list search algorithm, evolutionary programming, evolutionary strategy and neural networks.

18. The method according to claim 16, wherein the determining a plurality of candidate humanized antibody sequences and/or a plurality of candidate humanized antibody functional fragment sequences using a heuristic algorithm on the basis of the plurality of initial variable region sequences and the humanization probability comprises:
executing an iterative simulated evolution using a genetic algorithm on the basis of the plurality of initial variable region sequences and the humanization probability to determine a plurality of candidate variable region sequences; and
determining the plurality of candidate humanized antibody sequences and/or the plurality of candidate humanized antibody functional fragment sequences on the basis of the plurality of candidate variable region sequences.

19. The method according to claim 18, wherein the executing an iterative simulated evolution using a genetic algorithm on the basis of the plurality of initial variable region sequences and the humanization probability to determine a plurality of candidate variable region sequences comprises:
determining, for each of the plurality of initial variable region sequences, a distance between the initial variable region sequence and the target variable region sequence template, and determining an individual fitness of the initial variable region sequence on the basis of the distance between the initial variable region sequence and the target variable region sequence template and the humanization probability of the initial variable region sequence; and
executing an iterative simulated evolution on the basis of the plurality of initial variable region sequences and the individual fitness to determine a plurality of candidate variable region sequences.

20. The method according to claim 19, wherein the individual fitness is determined using an NGSA-II algorithm or a derivative algorithm thereof.

21. The method according to claim 19, wherein the target variable region sequence template comprises a target heavy chain variable region sequence template and a target light chain variable region sequence template, and the plurality of candidate variable region sequences comprises a plurality of candidate heavy chain variable region sequences and a plurality of candidate light chain variable region sequences.

22. The method according to claim 21, wherein the executing a simulated mutation on a framework region of the target variable region sequence template according to a preset mutation probability on the basis of the target variable region sequence template to obtain a plurality of initial variable region sequences comprises:
executing a simulated mutation on a framework region of the first variable region sequence template according to the preset mutation probability on the basis of a first variable region sequence template corresponding to a first variable region in the target variable region sequence template to obtain a plurality of initial populations corresponding to the first variable region, wherein each of the plurality of initial populations comprises the plurality of initial variable region sequences corresponding to the first variable region, and the first variable region is a heavy chain variable region or a light chain variable region.

23. The method according to claim 22, wherein each simulated evolution operation in the iterative simulated evolution comprises:
selecting a plurality of initial variable region sequences from the plurality of initial variable region sequences as a plurality of first sequences on the basis of the individual fitness of each of the plurality of initial variable region sequences corresponding to the first variable region;
retaining the plurality of first sequences of the plurality of initial populations and removing unselected initial variable region sequences of the plurality of initial populations to obtain a plurality of first populations;
executing a simulated migration among the plurality of first populations to obtain a plurality of second populations, each of the plurality of second populations comprising a plurality of second sequences;
executing a simulated crossover and a simulated variation on the plurality of second sequences of the plurality of second populations to obtain a plurality of third populations, each of the plurality of third populations comprising a plurality of third sequences; and
determining one or more first candidate variable region sequences from the plurality of third sequences.

24. The method according to claim 23, wherein a termination condition for the iterative simulated evolution comprises at least one of the following conditions:
a total number of rounds of the iterative simulated evolution being greater than or equal to a first threshold;
a total number of first candidate variable region sequences obtained by the iterative simulated evolution being greater than or equal to a second threshold;
a diversity of the one or more first candidate variable region sequences obtained by a current round of simulated evolution operation being less than or equal to a third threshold; and
an average similarity of first candidate variable region sequences obtained by a current round of simulated evolution operation to first candidate variable region sequences obtained by a previous round of simulated evolution operation being greater than or equal to a fourth threshold.

25. The method according to claim 23, wherein the first variable region sequence template comprises a first template and/or a second template, wherein the first template is a human-derived variable region sequence which is acquired from the database containing a plurality of human-derived variable region sequences and has the highest similarity to a sequence of a framework region of a first variable region of the initial antibody, and the second template is a human-derived variable region sequence which is acquired from the database containing a plurality of human-derived variable region sequences and has the highest similarity to a complete sequence of a first variable region of the initial antibody.

26. The method according to claim 25, wherein
the first variable region sequence template comprises the first template and the second template;
the plurality of initial populations comprises a plurality of first initial populations obtained by executing a simulated mutation on the basis of the first template and a plurality of second initial populations obtained by executing a simulated mutation on the basis of the second template; and
the plurality of first populations comprises a plurality of first retained populations obtained by retaining the first sequences from the first initial populations and a plurality of second retained populations obtained by retaining the first sequences from the second initial populations.

27. The method according to claim 26, wherein the executing a simulated migration among the plurality of first populations to obtain a plurality of second populations comprises:
executing a simulated migration among the plurality of first retained populations, executing a simulated migration among the plurality of second retained populations, and executing a simulated migration between the first retained populations and the second retained populations to obtain the plurality of second populations.

28. The method according to claim 21, wherein the determining a plurality of candidate humanized antibody sequences and/or a plurality of candidate humanized antibody functional fragment sequences on the basis of the plurality of candidate variable region sequences comprises:
determining a plurality of selected candidate heavy chain variable region sequences from the plurality of candidate heavy chain variable region sequences;
determining a plurality of selected candidate light chain variable region sequences from the plurality of candidate light chain variable region sequences; and
determining the plurality of candidate humanized antibody sequences and/or the plurality of candidate humanized antibody functional fragment sequences on the basis of the plurality of selected candidate heavy chain variable region sequences and the plurality of selected candidate light chain variable region sequences.

29. The method according to claim 15, wherein the trained deep forest model is obtained by the following steps:
acquiring a plurality of training samples corresponding to the first variable region, wherein each of the plurality of training samples comprises a human-derived or non-human-derived variable region sequence and a tag indicating that the sample variable region sequence is human-derived or non-human-derived, and the sample variable region sequence is subjected to length standardization processing; and
training an initial deep forest model using the plurality of training samples to obtain a trained deep forest model.

30. The method according to claim 15, wherein the performing sequence analysis on a sequence of an initial antibody to determine a variable region and a CDR region of the initial antibody comprises:
performing sequence analysis on the sequence of the initial antibody to determine a heavy chain variable region, a light chain variable region, a heavy chain CDR region, and a light chain CDR region of the initial antibody;
performing length standardization processing on a sequence of the heavy chain variable region; and
performing length standardization processing on a sequence of the light chain variable region.

31. A computing device, comprising at least one processor and at least one storage device, wherein the at least one storage device has stored thereon an instruction set used for designing a humanized antibody sequence, and the instruction set, when executed, causes the processor to execute the method according to any one of claims 1-30.

32. A non-transitory computer-readable storage medium, wherein the non-transitory computer-readable storage medium has stored thereon an instruction set used for designing a humanized antibody sequence, and the instruction set, when executed by a computing device, causes the computing device to execute the method according to any one of claims 1-30.
